# EUROPEAN PATENT APPLICATION

(11) **EP 4 198 061 A1**
(43) Date of publication of application: **21.06.2023**
(21) Application number: 22864785.5
(22) Date of filing: 28.02.2022
(51) Int. Cl.: C08B 37/16, A61K 31/724, A61P 3/06

(54) **PHARMACEUTICAL COMPOSITION COMPRISING GAMMA-CYCLODEXTRIN POLYMER AND USE THEREOF**

(30) Priority: 01.09.2021 KR 20210116499; 04.01.2022 KR 20220000933
(71) Applicant: Renatus Inc., Wonju-si, Gangwon-do 26421 (KR)
(72) Inventor: KIM, Hee Gon, Wonju-si, Gangwon-do 26421 (KR)
(74) Representative: Lavoix
(86) International application number: PCT/KR2022/002845
(87) International publication number: WO 2023/033276

(57) **Abstract**

Provided are: a pharmaceutical composition including a gamma-cyclodextrin-based polymer, for prevention and treatment of diseases related to cholesterol metabolism dysregulation; and use thereof. The pharmaceutical composition including a gamma-cyclodextrin polymer for prevention or treatment of diseases related to cholesterol metabolism dysregulation, according to an embodiment, by including the gamma-cyclodextrin-based polymer, may minimize extraction of plasma membrane cholesterol and thus not cause cytotoxicity and hemolytic activity, thereby facilitating intracellular metabolism and excretion of cholesterol, and effectively suppressing the secretion of IL-1β, MCP-1, and TNF-α cytokines, thereby exhibiting an excellent anti-inflammatory effect.

## Description

### TECHNICAL FIELD

One or more embodiments relate to a pharmaceutical composition including a gamma-cyclodextrin polymer for prevention or treatment of cholesterol metabolic diseases and a use thereof.

### BACKGROUND ART

Cyclodextrin has a cyclic structure consisting of glucopyranose units linked by α-(1,4) bonds, and includes α-cyclodextrin of six units, β-cyclodextrin of seven units, and γ-cyclodextrin of eight units. Since hydroxyl groups bonded to C₂ and C₃ of cyclodextrin and a hydroxyl group bonded to C₆ extend outward in opposite directions, the outer side of the ring is hydrophilic, and as hydrogen groups of C₃ and C₅ and ether-like oxygen are positioned inward, the inside of the ring is relatively hydrophobic. Cyclodextrin forms inclusion complexes by the inclusion of hydrophobic molecules in the ring, and due to its property of improving water-solubility of the accommodated hydrophobic molecules, cyclodextrin is applied in various fields, such as cosmetics, foods, textiles, environment and catalysts, drug delivery, and pharmaceuticals.

Although the heights of the ring structures of alpha-, beta-, and gamma-cyclodextrin are the same, the number of glucopyranose units thereof are different from one another, and thus the diameters and volumes inside the rings are different from one another. The inside of the ring of alpha-cyclodextrin having six glucopyranose units is the smallest, and the inside of the ring of gamma-cyclodextrin having eight glucopyranose units is the largest. Due to these differences, alpha-cyclodextrin is known to form complexes with relatively small molecules, and gamma-cyclodextrin with relatively large molecules in the ring.

Hearing loss caused by hydroxypropyl-beta-cyclodextrin, which is one of the most widely used derivatives of beta-cyclodextrin and in clinical trials as an active pharmaceutical ingredient, is known to be due to the toxicity to the outer hair cells of the cochlea. Also, it is known that cyclodextrin extracts cholesterol from the plasma membrane and causes cell membrane instability and collapse. Since the hair cells are highly sensitive to such plasma membrane disruption, cyclodextrin could lead to cytotoxicity and hearing loss. Therefore, there is a need to develop a drug capable of facilitating the removal of intracellular and extracellular cholesterol while not destroying plasma membrane, by minimizing the extraction of cholesterol from the plasma membrane.

### DESCRIPTION OF EMBODIMENTS

### TECHNICAL PROBLEM

According to an aspect of an embodiment, a pharmaceutical composition for prevention or treatment of diseases related to cholesterol metabolism dysregulation includes a gamma-cyclodextrin (γ-cyclodextrin) polymer or a derivative thereof.

According to another aspect of an embodiment, a food composition and a health functional food composition for prevention or alleviation of diseases related to cholesterol metabolism dysregulation includes a γ-cyclodextrin polymer or a derivative thereof.

According to another aspect of an embodiment, a method of preventing or treating diseases related to cholesterol metabolism dysregulation includes administering a γ-cyclodextrin polymer or a derivative thereof to a subject, excluding humans.

Other objects and advantages of the present disclosure will be apparent by the appended claims and the following detailed description together with the attached drawings. The contents that are not set forth in the specification can be sufficiently recognized and inferred by a person skilled in the art to which the present disclosure belongs or the art similar thereto, and descriptions thereof will be omitted.

### SOLUTION TO PROBLEM

Each of the explanations and exemplary embodiments disclosed herein may be applied to other explanations and exemplary embodiments. That is, all of the combinations of various factors disclosed herein belong to the scope of the present disclosure. Furthermore, the scope of the present disclosure should not be limited by the specific disclosure provided hereinbelow.

According to an aspect of an embodiment, provided is a pharmaceutical composition for prevention or alleviation of diseases related to cholesterol metabolism dysregulation including a gamma-cyclodextrin (γ-cyclodextrin) polymer having a molecular weight in a range of about 2.5 kDa to about 50 kDa or a derivative thereof.

The γ-cyclodextrin defined in the present specification is a cyclic oligosaccharide in which glucopyranose units each represented by Formula 1 are bonded by α-(1,4) bonds and may include gamma-cyclodextrin of eight units and a derivative thereof.

As used herein, the term "derivative" refers to a compound obtained by substituting a part of a structure of the compound, particularly, a hydroxyl group of C₂, C₃, or C₆, with another atom or atomic group.

A weight average molecular weight (hereinafter, also referred to as "molecular weight") of the gamma-cyclodextrin-based polymer may be in a range of, for example, about 2.5 kDa to about 50 kDa, about 3 kDa to about 50 kDa, about 3.5 kDa to about 50 kDa, about 4 kDa to about 50 kDa, about 4.5 kDa to about 50 kDa, about 5 kDa to about 50 kDa, about 5.5 kDa to about 50 kDa, about 6 kDa to about 50 kDa, about 2.5 kDa to about 45 kDa, about 3 kDa to about 45 kDa, about 3.5 kDa to about 45 kDa, about 4 kDa to about 45 kDa, about 4.5 kDa to about 45 kDa, about 5 kDa to about 45 kDa, about 5.5 kDa to about 45 kDa, about 6 kDa to about 45 kDa, about 2.5 kDa to about 40 kDa, about 3 kDa to about 40 kDa, about 3.5 kDa to about 40 kDa, about 4 kDa to about 40 kDa, about 4.5 kDa to about 40 kDa, about 5 kDa to about 40 kDa, about 5.5 kDa to about 40 kDa, about 6 kDa to about 40 kDa, about 2.5 kDa to about 35 kDa, about 3 kDa to about 35 kDa, about 3.5 kDa to about 35 kDa, about 4 kDa to about 35 kDa, about 4.5 kDa to about 35 kDa, about 5 kDa to about 35 kDa, about 5.5 kDa to about 35 kDa, about 6 kDa to about 35 kDa, about 2.5 kDa to about 30 kDa, about 3 kDa to about 30 kDa, about 3.5 kDa to about 30 kDa, about 4 kDa to about 30 kDa, about 4.5 kDa to about 30 kDa, about 5 kDa to about 30 kDa, about 5.5 kDa to about 30 kDa, about 6 kDa to about 30 kDa, about 2.5 kDa to about 25 kDa, about 3 kDa to about 25 kDa, about 3.5 kDa to about 25 kDa, about 4 kDa to about 25 kDa, about 4.5 kDa to about 25 kDa, about 5 kDa to about 25 kDa, about 5.5 kDa to about 25 kDa, about 6 kDa to about 25 kDa, about 2.5 kDa to about 20 kDa, about 3 kDa to about 20 kDa, about 3.5 kDa to about 20 kDa, about 4 kDa to about 20 kDa, about 4.5 kDa to about 20 kDa, about 5 kDa to about 20 kDa, about 5.5 kDa to about 20 kDa, about 6 kDa to about 20 kDa, about 6.5 kDa to about 50 kDa, about 6.5 kDa to about 45 kDa, about 6.5 kDa to about 40 kDa, about 6.5 kDa to about 30 kDa, about 6.5 kDa to about 20 kDa, about 7 kDa to about 50 kDa, about 7 kDa to about 45 kDa, about 7 kDa to about 40 kDa, about 7 kDa to about 30 kDa, about 7 kDa to about 20 kDa, about 7.5 kDa to about 50 kDa, about 7.5 kDa to about 45 kDa, about 7.5 kDa to about 40 kDa, about 7.5 kDa to about 30 kDa, about 7.5 kDa to about 20 kDa, about 8 kDa to 50 kDa, about 8 kDa to 45 kDa, about 8 kDa to 40 kDa, about 8 kDa to 30 kDa, about 8 kDa to 20 kDa, about 2.5 kDa to about 20 kDa, about 2.6 kDa to about 20 kDa, about 4 kDa to about 20 kDa, or about 10 kDa to about 20 kDa.

When the molecular weight of the gamma-cyclodextrin-based polymer is out of these ranges, its effect on increasing the levels of ABCA1 and cholesteryl ester in cells may decrease, anti-inflammatory effects may decrease when used alone or in combination with other drugs, and excreting cholesterol from the body may be reduced.

The gamma-cyclodextrin-based polymer refers to a polymer formed of at least two gamma-cyclodextrin monomers that are linked by a covalent bond, and, for example, a bifunctional cross-linker such as epichlorohydrin may be used for the cross-linking of the monomers, but embodiments are not limited thereto.

The number of cyclodextrin monomers consisting the gamma-cyclodextrin polymer may be in a range of, for example, about 2 to 35, about 2 to 30, about 2 to 25, about 2 to 20, about 2 to 15, about 2 to 10, about 3 to 35, about 3 to 30, about 3 to 25, about 3 to 20, about 3 to 15, about 3 to 10, about 4 to 35, about 4 to 30, about 4 to 25, about 4 to 20, about 4 to 15, about 4 to 10, about 5 to 35, about 5 to 30, about 5 to 25, about 5 to 20, about 5 to 15, or about 5 to 10.

As used herein, a derivative may be induced by replacing at least one hydrogen in an unsubstituted mother group with another atom or a functional group. Unless stated otherwise, when a functional group is deemed as "substituted", it means that the functional group is substituted with at least one substituent selected from halides, C1-C40 alkyl groups, C2-C40 alkenyl groups, C2-C40 alkynyl groups, C3-C40 cycloalkyl groups, C3-C40 cycloalkenyl groups, and C7-C40 aryl groups.

When it is stated as a functional group is "selectively substituted", it means that the functional group may be substituted with the above substituent.

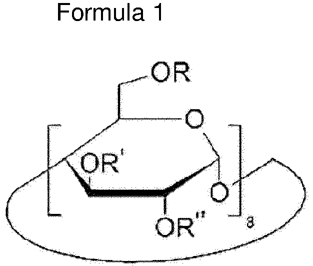

The cyclodextrin polymer according to an embodiment may be a polymer having a substituent, and the cyclodextrin polymer according to an embodiment may be a gamma-cyclodextrin polymer represented by Formula 1. R, R' and R" bonded to hydroxyl groups of C₂, C₃, and C₆ of Formula 1 may be, for example, hydrogen, C1-C10 linear or branched alkyl, hydroxy C1-C10 linear or branched alkyl, sulfobutylether C1-C10 linear or branched alkyl, or carboxy C1-C10 linear or branched alkyl; particularly, may be hydrogen, C1-C5 linear or branched alkyl, hydroxy C1-C5 linear or branched alkyl, sulfobutylether C1-C5 linear or branched alkyl, or carboxy C1-C5 linear or branched alkyl; or more particularly, may be hydrogen, methyl, hydroxypropyl, sulfobutylether, or carboxy methyl, but embodiments are not limited thereto.

In the gamma-cyclodextrin derivative of Formula 1, the number of the substituted hydroxy groups may be, for example, 1 to 24, 1 to 18, 1 to 12, 2 to 24, 2 to 18, 3 to 24, 3 to 12, 4 to 6, or about 5, but embodiments are not limited thereto.

When the substituted hydroxyl group per one glucose is shown as a molar substitution, the molar substitution of the gamma-cyclodextrin derivative may be, for example, about 0.125 to about 3, about 0.125 to about 2.25, about 0.125 to about 1.5, about 0.25 to about 3, about 0.25 to about 2.25, about 0.375 to about 3, about 0.375 to about 1.5, about 0.5 to about 0.75, or around 0.6 or so, but embodiments are not limited thereto.

In the present specification, the gamma-cyclodextrin polymer may be a polymer of hydroxypropyl-gamma-cyclodextrin represented by Formula 2 having a molar substitution in a range of about 0.5 to about 0.75.

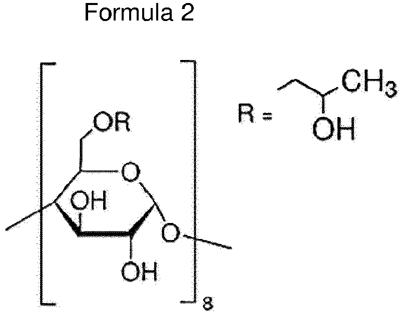

In the polymer of hydroxypropyl-gamma-cyclodextrin of Formula 2, the degree of molar substitution of the polymer may be in a range of, for example, about 0.125 to about 3, about 0.125 to about 2.25, about 0.125 to about 1.5, about 0.25 to about 3, about 0.25 to about 2.25, about 0.375 to about 3, about 0.375 to about 1.5, about 0.5 to about 0.75, or about around 0.6, but embodiments are not limited thereto.

In the present specification, the gamma-cyclodextrin polymer may be a polymer of hydroxypropyl-gamma-cyclodextrin represented by Formula 2 having a molar substitution in a range of about 0.5 to about 0.75, and in one embodiment, the cyclodextrin was dissolved in 33 % NaOH in a concentration of 500 mg/mL, 350 mg/mL, 250 mg/mL, and 125 mg/mL, and polymerization was performed using molar ratios of epichlorohydrin verses cyclodextrin (epichlorohydrin/cyclodextrin) of 0.5, 1, 3, 5, 7, and 10, respectively, to prepare the hydroxypropyl-gamma-cyclodextrin. In particular, it was confirmed that in the concentration of 250 mg/mL, a water-soluble polymer having a molecular weight of about 5.3 kDa to about 72.8 kDa was prepared depending on the molar ratio of epichlorohydrin to cyclodextrin, and thus it was confirmed that a water-soluble hydroxypropyl-gamma-cyclodextrin polymer of an appropriate size (molecular weight) may be prepared by controlling the concentration of cyclodextrin and the molar ratio of epichlorohydrin.

Cross-linking of hydroxypropyl-gamma-cyclodextrin by epichlorohydrin may occur, for example, between alkoxide-induced hydroxyl groups, between a hydroxyl group and a hydroxypropyl group, and between hydroxypropyl groups, and an example thereof is shown in the following Reaction Scheme 1.

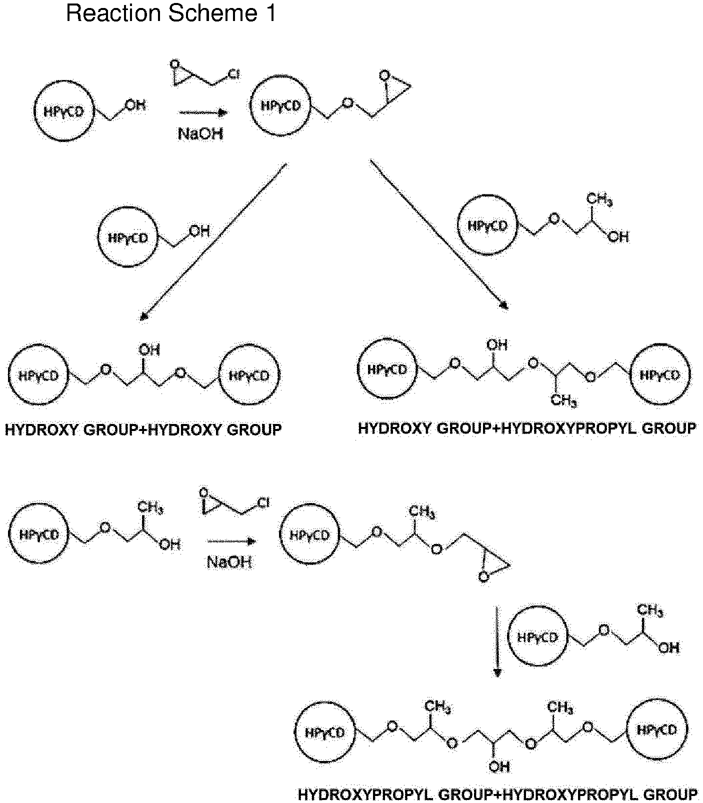

Also, the hydroxypropyl-gamma-cyclodextrin may have several alkoxide groups per one monomer, among which random cross-linking occurs, and thus the hydroxypropyl-gamma-cyclodextrin may have a linear or cyclic composite structure. Examples of the structure of the polymer that may be formed are as shown in Table 1, but embodiments are not limited thereto.

**[Table 1]**

| **Number of cyclodextrin** | **Example of polymer structure** |
|---|---|
| 2 | 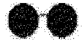 |
| 3 | 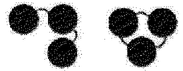 |
| 4 | 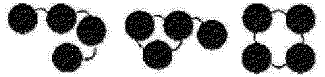 |
| 5 | 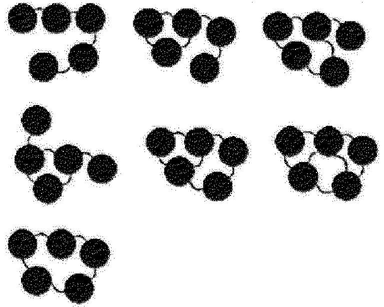 |

As used herein, the term "prevention" refers to all actions that suppress diseases related to cholesterol metabolism dysregulation or delay the onset thereof by administration of the pharmaceutical composition according to the present disclosure.

As used herein, the term "treatment" refers to all actions that alleviate or beneficially change symptoms of diseases related to cholesterol metabolism dysregulation by administration of the pharmaceutical composition according to the present disclosure.

As used herein, the term "alleviation" refers to all actions that at least reduce a parameter of diseases related to cholesterol metabolism dysregulation.

As used herein, the term "subject" refers to a target in need of treatment of diseases, and more specifically, a mammal such as a human or a non-human primate, a rodent (e.g., a rat, a guinea pig, etc.), a mouse, a dog, a cat, a horse, a cow, a sheep, a pig, a goat, a camel, or an antelope.

The diseases related to cholesterol metabolism dysregulation may include diseases that occur by abnormal cholesterol metabolism accumulating cholesterol in various tissued in the body such as blood vessels, brain, kidney, liver, lung, etc. of the body, as normal cholesterol metabolism does not appear, cholesterol dissolution does not appear outside the cell, and intracellular cholesterol metabolism and excretion do not appear. The diseases related to cholesterol metabolism dysregulation may include, for example, those selected from the group consisting of Niemann-pick disease type C, Alzheimer's dementia, Parkinson's disease, focal segmental glomerulosclerosis (FSGS), Alport syndrome, diabetic kidney disease, multiple sclerosis, interstitial pneumonia, osteoarthritis, dyslipidemia, and cardiovascular diseases caused by cholesterol homeostasis dysregulation.

The "dyslipidemia" refers to a disease caused by all lipid abnormalities resulting from an increase in low-density lipoprotein cholesterol (LDL-C) and triglycerides (TG) and a decrease in high-density lipoprotein cholesterol (HDL-C). In particular, the dyslipidemia may be at least one selected from the group consisting of hyperlipidemia, hypercholesterolemia, hypertriglyceridemia, combined hyperlipidemia, and combined dyslipidemia, but elements are not limited thereto.

The "hyperlipidemia" refers to a disease caused by an increase in lipids and/or lipoproteins in the blood to an abnormal level. Hyperlipidemia may include hypercholesterolemia, hypertriglyceridemia, and combined hyperlipidemia, according to the type of the increased lipid. The "dyslipidemia" refers to a state of the total cholesterol in the blood has an increase in LDL cholesterol (LDL-C) and triglycerides (TG) and a decrease in HDL cholesterol (HDL-C).

The cardiovascular diseases caused by cholesterol metabolism dysregulation may be, for example, at least one selected from the group consisting of coronary artery disease, peripheral vascular disease, atherosclerotic cardiovascular disease, and cerebrovascular disease, or particularly, for example, at least one selected from the group consisting of hypertension, angina, myocardial infarction, cerebral infarction, stroke, arrhythmia, dyslipidemia, hyperlipidemia, coronary arteriosclerosis, atherosclerosis, hypercholesterolemia, arteriosclerosis, atherosclerosis, hypertension, and angina.

The pharmaceutical composition may further include a pharmaceutical additive selected from the group consisting of a diluent, a binder, a disintegrant, a lubricant, and any combination thereof other than the gamma-cyclodextrin polymer. Also, the pharmaceutical composition may be formulated into injections such as aqueous solutions, suspensions, emulsions, etc., pills, capsules, granules or tablets, with the aid of a diluent, a dispersant, a surfactant, a binder, and/or a lubricant. Also, the pharmaceutical composition may further include a pharmaceutically acceptable carrier, i.e., saline, sterile water, Ringer's solution, buffered saline, cyclodextrin, a dextrose solution, a maltodextrin solution, glycerol, ethanol, liposome, or a mixture of one or more thereof, and if necessary, other common additive such as an antioxidant, a buffer, etc. Moreover, the pharmaceutical composition may be formulated according to respective components using an appropriate method known in the art or a method disclosed in Remington's Pharmaceutical Science (Mack Publishing Company, Easton PA).

The diluent, which may be used to increase quantity, may be selected from the group consisting of mannitol, lactose, starch, microcrystalline cellulose, Ludipress^{®}, calcium dihydrogen phosphate, and any combinations thereof, but embodiments are not limited thereto.

The binder may be selected from the group consisting of povidone, hydroxypropyl cellulose, hydroxypropyl methyl cellulose, polyvinyl alcohol, sodium carboxymethyl cellulose, and any combinations thereof, but embodiments are not limited thereto.

The disintegrant may be selected from the group consisting of croscarmellose sodium, crospovidone, sodium starch glycolate, and any combinations thereof, but embodiments are not limited thereto.

The lubricant may be selected from the group consisting of stearic acid, metal salts of stearic acid (for example, calcium stearate, or magnesium stearate), talc, colloid silica, sucrose fatty acid ester, hydrogenated vegetable oil, wax, glyceryl fatty acid esters, glycerol dibehenate, and any combinations thereof, but embodiments are not limited thereto.

In the pharmaceutical composition, the gamma-cyclodextrin polymer or a derivative thereof may be included in an amount sufficient to achieve the efficacy or activity. One of ordinary skill in the art may select the upper limit and lower limit of the quantity of the compound contained in the composition within an appropriate range. The pharmaceutical composition may be administered in an dose of, for example, about 0.0001 mg/kg to about 1000 mg/kg, or, to be more effective, about 0.01 mg/kg to about 100 mg/kg, based on the pharmaceutical composition.

The pharmaceutical composition may be for oral or parenteral administration, and the pharmaceutical composition may be administered orally or parenterally. The pharmaceutical composition may be formulated into an oral or parenteral formulation.

When the pharmaceutical composition is prepared into an oral formulation, the oral formulation may be prepared based on a method for preparing any oral solid dosage as known in the art, specifically, a granule, a pellet, a capsule, or a tablet. The oral formulation may include granules, powders, solutions, tablets, capsules, dry syrups, or combinations thereof. The parenteral formulation may include injectable formulations or external preparations for skin. The external preparations for skin may include creams, gels, ointments, skin emulsions, skin suspensions, transdermal patches, drug-containing bandages, lotions, or combinations thereof. Parenteral administration may include, for example, intravenous injection, subcutaneous injection (or administration), muscular injection, intraperitoneal injection, endothelial administration, local administration, intranasal administration, intrapulmonary administration, intracerebral administration, and rectal administration. For example, the pharmaceutical composition may be administered intravenously or subcutaneously for systemic deliver of the composition, and may be administered intrathecally for central nervous system delivery of the pharmaceutical composition. Also, the composition may be administered by any device which can transport active substances to target cells. A dose of the pharmaceutical composition according to an embodiment may vary depending on the patient's weight, age, sex, state of health and diet, the duration of administration, the mode of administration, excretion rate, or severity of disease, and may be easily determined by those skilled in the art. In addition, for clinical administration, the composition of the present disclosure may be prepared into a suitable formulation using a known technique.

The dose of the composition of the present disclosure may be determined by a person skilled in the art based on the condition of the patient and the severity of the disease. In addition, the composition may be formulated in various dosage forms, including powders, tablets, capsules, liquids, injectable solutions, ointments, and syrup formulations, and may be provided in unit-dosage or multi-dosage containers, for example, sealed ampules or vials.

Also, the pharmaceutical composition according to an embodiment may be administered in combination with another drug sequentially or simultaneously. The composition according to an embodiment may be administered in combination with, for example, at least one selected from the group consisting of a liver X receptor (LXR) agonist, a GLP-1 receptor, an ezetimibe agent, a fibric acid derivative, a nicotinic acid-based agent, a PCSK9 inhibitor, and an HMG-CoA reductase inhibitor.

The LXR agonist may be, for example, selected from the group consisting of 22(R)-hydroxycholesterol, 24(S),25-epoxycholesterol, 24(S)-hydroxycholesterol, 27-hydroxycholesterol, cholesteroic acid, T-314407, T-0901317, GW3965, SB742881 , GSK 2186, WAY-252623 (LXR-623), WAY-254011, WYE-672, N,N-Dimethyl-3b-hydroxycholenamide (DMHCA), 4-(3-biaryl)quinoline sulfone, F₃MethylAA, acetyl-podocarpic dimer (APD), podocarpic imide dimer, CRX000541, CRX000864, CRX000929, CRX000823, CRX000987, CRX001093, CRX001094, CRX156651, CRX000909, CRX000908, CRX000369, CRX001045, CRX001046, LN7181, LN7179, LN7172, LN6672, LN7031, LN7033, LN6500, LN6662, Riccardin C, XL-652, DY136, DY142, and hypocholamide (3a,6a-dihydroxy-5b-cholanoic acid-N-methyl-N-methoxy-24- amide).

The ezetimibe agent may be ezetimibe or a pharmaceutically acceptable salt thereof.

The GLP-1 receptor may be, for example, selected from the group consisting of exenatide, lixisenatide, and liraglutide.

The fibric acid derivative may be, for example, selected from the group consisting of gemfibrozil, bezafibrate, fenofibrate, and ciprofibrate.

The nicotinic acid-based agent may be, for example, acipimox.

The HMG-CoA reductase inhibitor may be, for example, selected from the group consisting of rosuvastatin, simvastatin, atorvastatin, pitavastatin, pravastatin, fluvastatin, and lovastatin.

The pharmaceutical composition according to an embodiment has less properties of causing cell membrane cholesterol extraction, which lowers cytotoxicity and hemolytic activity, has excellent cholesterol-solubilizing ability, and is capable of improving cholesterol metabolism in cells. Also, it is possible to exhibit anti-inflammatory activity by alleviating the inflammatory response of cells through the regulation of cholesterol metabolism, thereby reducing the secretion of inflammatory cytokines. Also, the gamma-cyclodextrin polymer in the pharmaceutical composition according to an embodiment has characteristics of improving cholesterol metabolism and excretion when injected into the body and not exhibiting ototoxicity even at high concentrations.

According to another aspect of an embodiment, provided is a food composition or a health functional food composition for prevention or alleviation of diseases related to cholesterol metabolism dysregulation, the composition including a gamma-cyclodextrin polymer having a molecular weight of about 2.5 kDa to about 50 kDa or a polymer including a derivative thereof.

In the present specification, food refers to natural or processed food which contains at least one or more nutrients, preferably, represents an eatable thing somehow processed, and as an ordinary meaning, it represents foods, food additives, health functional foods, and beverage.

The foods to which the gamma-cyclodextrin polymer according to an embodiment may be added, for example, are various foods, beverages, gums, candies, teas, vitamin mixtures, functional foods, etc. In addition, the foods of the present disclosure include special nutrition foods (for example, milk formulas or infants and kids' foods), eatable processed foods, fish cakes, tofu, starch gel, noodle (for example, ramen or noodle), health supplemental foods, flavoring foods (for example, soy sauce, soy bean paste, red pepper paste, or mixed paste), sauces, cookies (for example, snacks), milk products (for example, fermented oil or cheese), other processed foods, kimchi, salt soaked foods (various kimchi or soy bean-soaked vegetable), beverage (for example, fruit or vegetable beverage, tofu, fermented beverage, or ice cream), natural flavoring sauces (for example, dried ramen soup), vitamin mixtures, alcohol beverage, drinks, and other health supplemental foods, but embodiments are not limited thereto. The foods, beverage, or food additives may be manufactured according to an ordinary method.

In the present specification, the functional food represents a food group valueadded to make sure that the function of a corresponding food may be more promoted from its original function with the aid of a physical, biochemical, or biological engineering method or a processed food which is designed to have more of the body control function such as a biological prevention rhythm, a disease prevention and recovery that a food composition has. Preferably, the functional food may refer to a food capable of sufficiently exhibiting the body control function in relation to prevention or alleviation of diseases related to cholesterol metabolism dysregulation. The functional food may include a food supplemental additive which is allowable on the basis of food science and may further include an appropriate carrier, an excipient, and a diluent that are generally used in preparation of functional foods.

A mixing amount of active ingredients may be suitably determined depending on the purpose of use, for example, disease prevention or therapeutic treatment. Generally, when manufacturing foods or beverages, the gamma-cyclodextrin may be added to food alone or in combination with other food or food component, and the mixing amount of the gamma-cyclodextrin polymer may be appropriately determined according to a purpose of use thereof (for prevention or alleviation). In food or beverage manufacture, the gamma-cyclodextrin polymer may be added in an amount of about 15 parts by weight or less, about 10 parts by weight or less, about 1 part to about 15 parts by weight, about 1 part to about 10 parts by weight, about 1 part to about 5 parts by weight, or about 0.1 parts to about 15 parts by weight, based on the total weight of the composition. However, when consumed for a long period of time for health and sanitary purposes, the composition may be used in an amount below these ranges. Also, because the active ingredient carries no safety risk, the active ingredient may be used in an amount above these ranges.

The food may be formulated into at least one selected from the group consisting of tablets, pills, powders, granules, fine powders, capsules, and solutions by further including one or more of carriers, excipients, diluents, and additives.

Examples of the carriers, excipients, diluents, and additives may be at least one selected from the group consisting of lactose, dextrose, sucrose, sorbitol, mannitol, erythritol, starch, acacia rubber, calcium phosphate, alginate, gelatin, calcium phosphate, calcium silicate, microcrystalline cellulose, polyvinylpyrrolidone, cellulose, polyvinylpyrrolidone, methylcellulose, water, sugar syrup, methylcellulose, methylhydroxy benzoate, propylhydroxy benzoate, talc, magnesium stearate, and mineral oil.

The "health functional food" defined in the present specification refers to foods produced or processed using raw materials or components that have useful functionality in the human body according to the health functional food law, and the term "functional" means that the food is ingested for the purpose of obtaining a beneficial effect for health use such as controlling nutrients or physiological action for the structure and function of the human body.

The health functional food for preventing or alleviating diseases related to cholesterol metabolism dysregulation according to an embodiment may include about 0.01 % to about 95 %, preferably, about 1 % to about 80 %, of the gamma-cyclodextrin or a derivative thereof, based on the total weight of the composition. Also, the health functional food may be produced or processed in the form of tablets, capsules, powders, granules, solutions, or pills in the purpose of preventing or alleviating diseases related to cholesterol metabolism dysregulation.

According to another aspect of an embodiment, a method of preventing or treating diseases related to cholesterol metabolism dysregulation includes administering a gamma-cyclodextrin polymer or a derivative thereof to a subject, excluding humans.

A dose of the gamma-cyclodextrin polymer according to an embodiment may be determined in consideration of dosage method, age and sex of takers, severity and conditions of patients, intake of active ingredient in the body, inactivation ratio, and drugs used in conjunction therewith. Based on the active ingredient per day, a dose may be, for example, about 10 mg/kg to about 10,000 mg/kg, about 100 mg/kg to about 5000 mg/kg, or about 500 mg/kg to about 2500 mg/kg for systemic delivery, and may be, for example, about 10 mg to about 5,000 mg, about 50 mg to about 4,000 mg, or about 100 mg to about 3,000 mg for central nervous system direct delivery. The composition may be administered once or in several portions, but embodiments are not limited thereto.

The gamma-cyclodextrin polymer of the present disclosure may be used alone or in combination with surgery, radiation therapy, chemotherapy, and methods using biological response modifiers for treatment and prevention of diseases related to cholesterol metabolism dysregulation.

### ADVANTAGEOUS EFFECTS OF DISCLOSURE

The pharmaceutical composition including a gamma-cyclodextrin (γ-cyclodextrin) polymer for prevention or treatment of diseases related to cholesterol metabolism dysregulation according to an embodiment may minimize extraction of plasma membrane cholesterol by including the gamma-cyclodextrin-based polymer, which does not cause cytotoxicity and hemolytic activity to facilitate metabolism and excretion of cholesterol in cells, and effectively suppresses secretion of IL-1β, MCP-1, and TNF-α cytokines, thereby exhibiting an excellent anti-inflammatory effect. Also, when administered in combination with other drugs, the gamma-cyclodextrin polymer may exhibit excellent cholesterol excretion and anti-inflammatory effect, as compared to those of other cyclodextrin polymers.

Also, the gamma-cyclodextrin polymer may be effectively excreted to the outside of the body through glomerular filtration, and even when injected at high doses, hearing loss and systemic side effects do not occur, and thus the polymer may be used in prevention or treatment of diseases related to cholesterol metabolism dysregulation such as arteriosclerosis, Alzheimer's dementia, focal segmental glomerulosclerosis, and Niemann-Pick's disease type C.

### BRIEF DESCRIPTION OF DRAWINGS

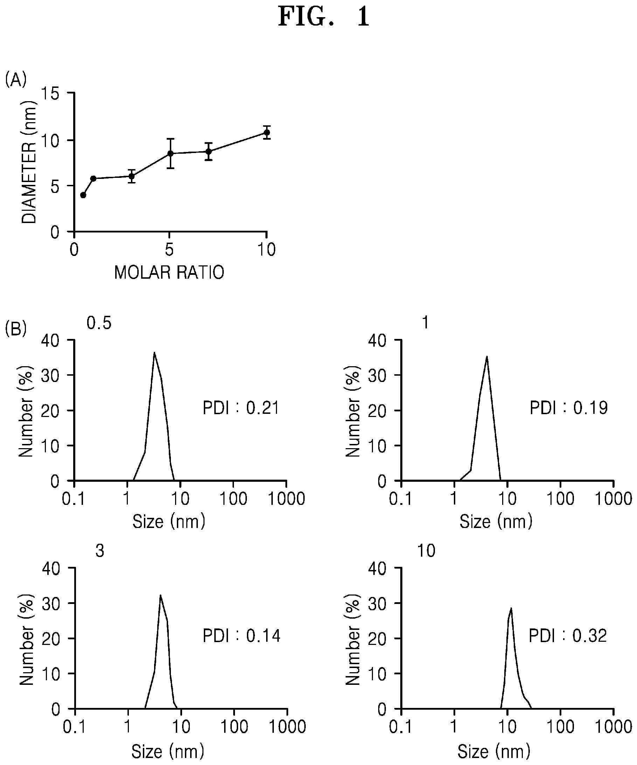

FIG. 1 shows the hydrodynamic diameters of polymers formed at a cyclodextrin concentration of 250 mg/mL (FIG. 1A) and the representative size distribution when the molar ratios of epichlorohydrin to hydroxypropyl-gamma-cyclodextrin are 0.5, 1, 3, and 10 (FIG. 1B). Data are expressed as mean ± standard deviation (SD) (where n of A = 3);

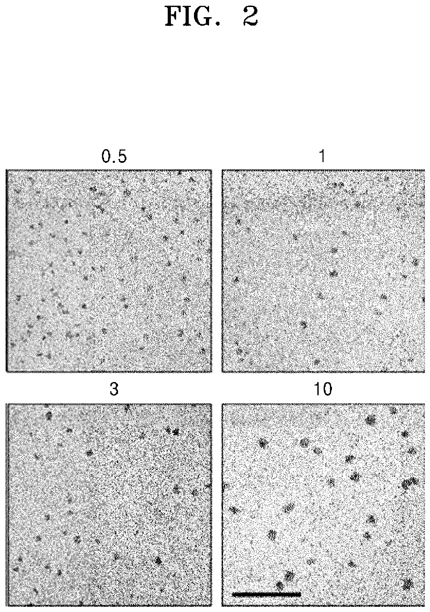

FIG. 2 shows representative images of hydroxypropyl-gamma-cyclodextrin polymer as observed by scanning electron microscopy when the molar ratios of epichlorohydrin to hydroxypropyl-gamma-cyclodextrin are 0.5, 1, 3, and 10 (where scale bars are 100 nm);

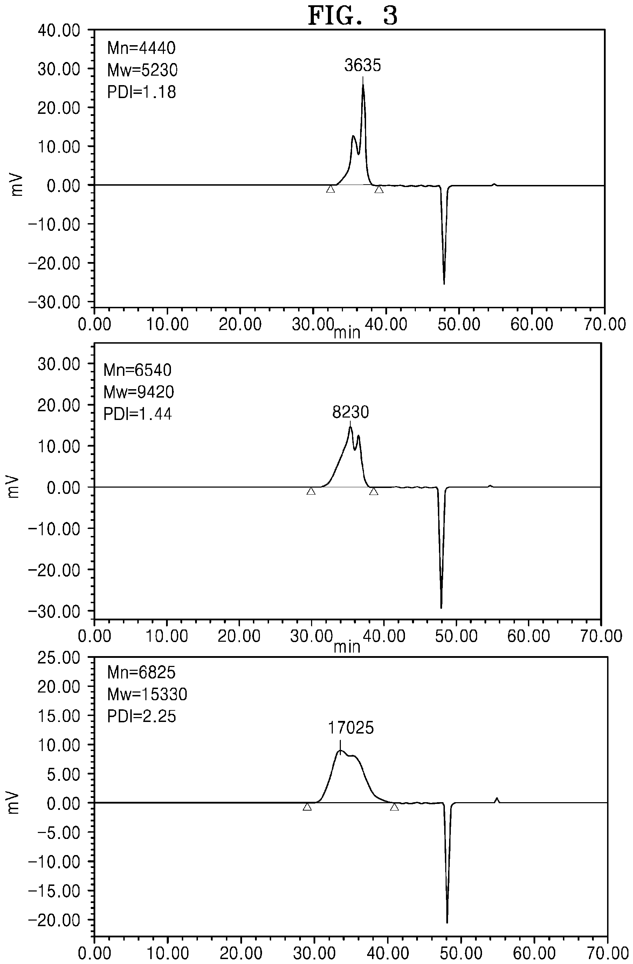

FIG. 3 shows representative gel permeation chromatographs of hydroxypropyl-gamma-cyclodextrin polymer when molar ratios are 0.5 (top), 1 (middle), and 3 (bottom);

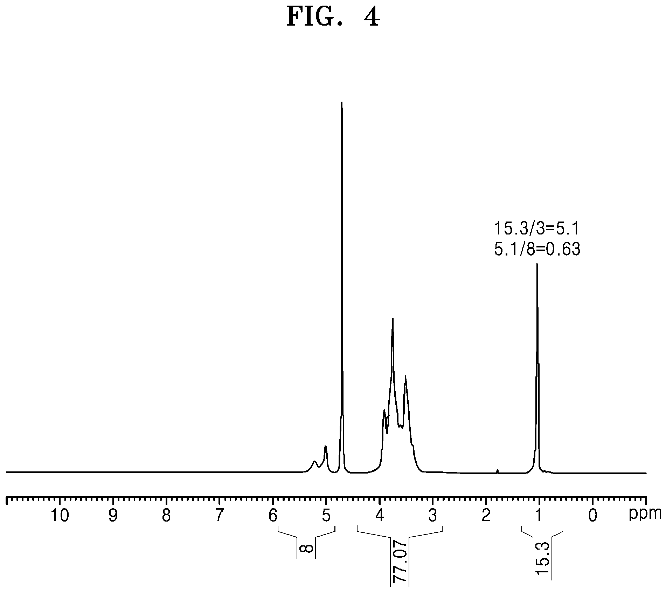

FIG. 4 shows a nuclear magnetic resonance (NMR) analysis of hydroxypropyl-gamma-cyclodextrin polymer (where an average molar substitution is 0.63);

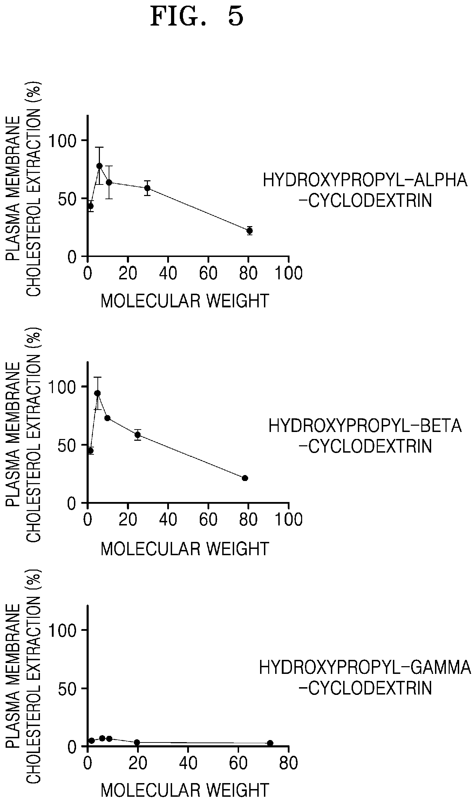

FIG. 5 shows the level of plasma membrane cholesterol extraction depending on the molecular weights of monomers and polymers of hydroxypropyl-alpha-cyclodextrin (top), -beta-cyclodextrin (middle), and -gamma-cyclodextrin (bottom). Data are expressed as mean ± SD (where n = 3);

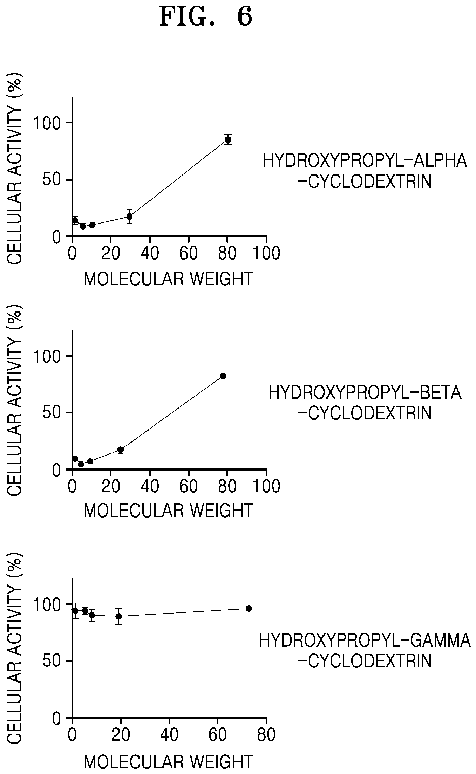

FIG. 6 shows the level of cell viability depending on the molecular weights of monomers and polymers of hydroxypropyl-alpha-cyclodextrin (top), -beta-cyclodextrin (middle), and -gamma-cyclodextrin (bottom). Data are expressed as mean ± SD (where n = 3);

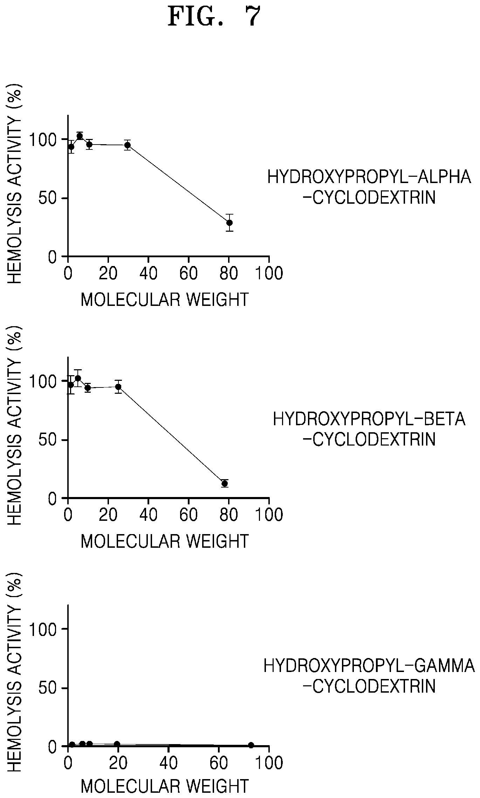

FIG. 7 shows the level of hemolytic activity depending on the molecular weights of monomers and polymers of hydroxypropyl-alpha-cyclodextrin (top), -beta-cyclodextrin (middle), and -gamma-cyclodextrin (bottom). Data are expressed as mean ± SD (where n = 3);

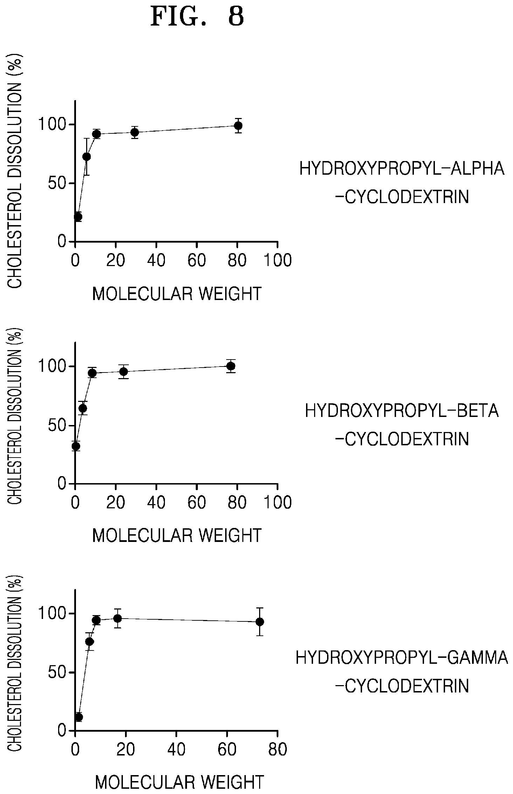

FIG. 8 shows the level of cholesterol crystal dissolution depending on the molecular weights of monomers and polymers of hydroxypropyl-alpha-cyclodextrin (top), -beta-cyclodextrin (middle), and -gamma-cyclodextrin (bottom). Data are expressed as mean ± SD (where n = 3);

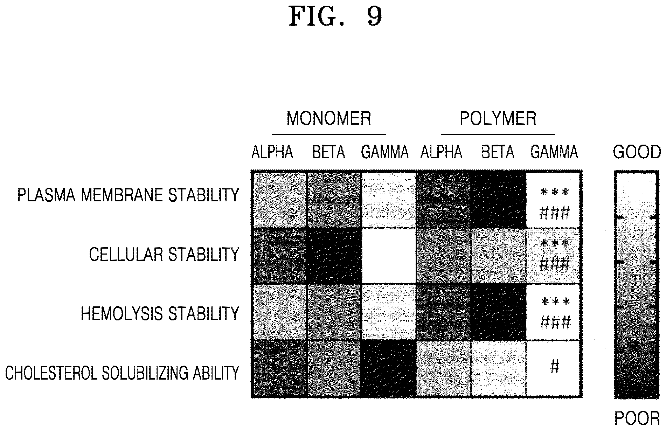

FIG. 9 shows a heat map of plasma membrane stability, cell stability, hemolytic stability, and cholesterol-solubilizing ability of hydroxypropyl-alpha-, -beta-, -gamma-cyclodextrin monomers and polymers. ***P < 0.001 is a value obtained by comparing with hydroxypropyl-alpha-cyclodextrin polymer, #P < 0.05 and ###P < 0.001 are values that are obtained by comparing with hydroxypropyl-beta-cyclodextrin polymer, and One-way ANOVA analysis and Tukey's post-hoc analysis were used. Data are expressed as mean ± SD (where n = 3);

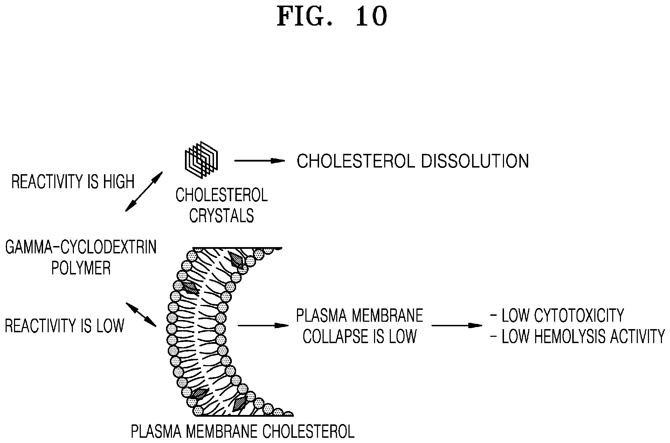

FIG. 10 is a schematic view illustrating the features of hydroxypropyl-gamma-cyclodextrin polymer;

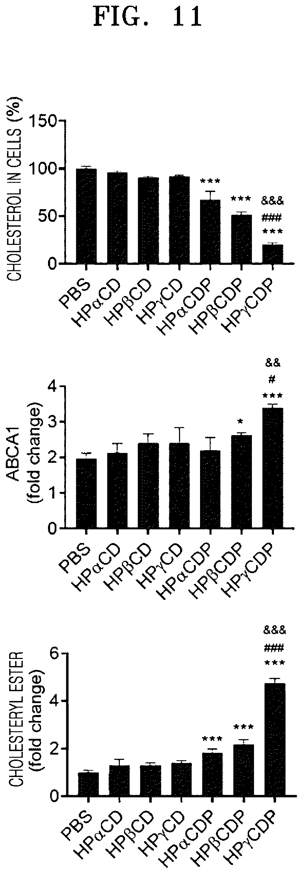

FIG. 11 shows the levels of cholesterol (top), ABCA1 (middle), and cholesteryl ester (bottom) that remained in the cells after macrophages that had taken up fluorescent cholesterol were treated with cyclodextrin. *P < 0.05 and ***P < 0.001 are values obtained by comparing with phosphate-buffered saline (PBS), #P < 0.05 and ###P < 0.001 are values obtained by comparing with hydroxypropyl-beta-cyclodextrin polymer, &&P < 0.01 and &&&P < 0.001 are values obtained by comparing with hydroxypropyl-alpha-cyclodextrin polymer, and One-way ANOVA analysis and Tukey's post-hoc analysis were used. Data are expressed as mean ± SD (where n = 3). HPαCDP represents the hydroxypropyl-alpha-cyclodextrin polymer, HPβCDP represents the hydroxypropyl-beta-cyclodextrin polymer, and HPγCDP represents the hydroxypropyl-gamma-cyclodextrin polymer;

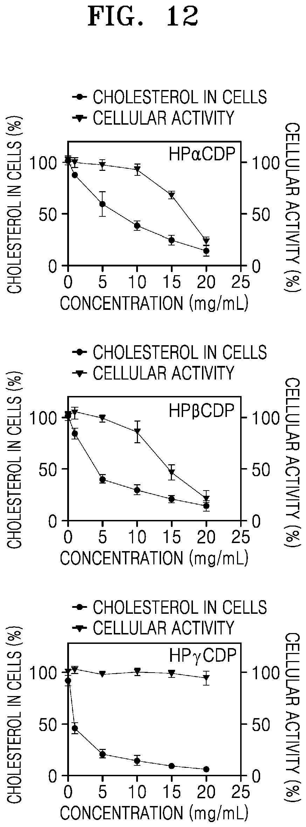

FIG. 12 shows the amounts of cholesterol that remained in the cells after macrophages that had taken up fluorescent cholesterol were treated with cyclodextrin polymers (HPαCDP: top, HPβCDP: middle, and HPγCDP: bottom) at various concentrations, and the cell viability;

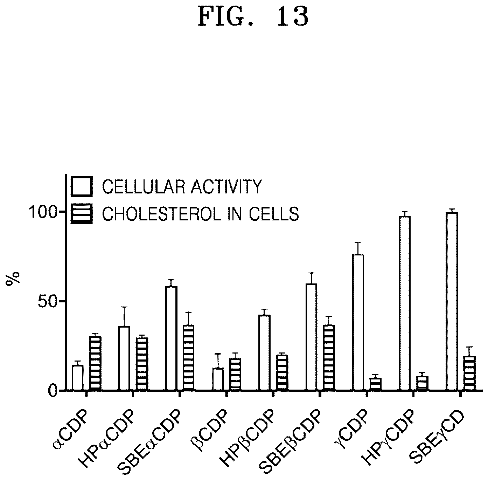

FIG. 13 is a graph of the amounts of cholesterol remaining in the cells after macrophages that had taken up fluorescent cholesterol were treated with gamma-cyclodextrin polymer and sulfobutylether-gamma-cyclodextrin polymer in various concentrations, and the cellular activity;

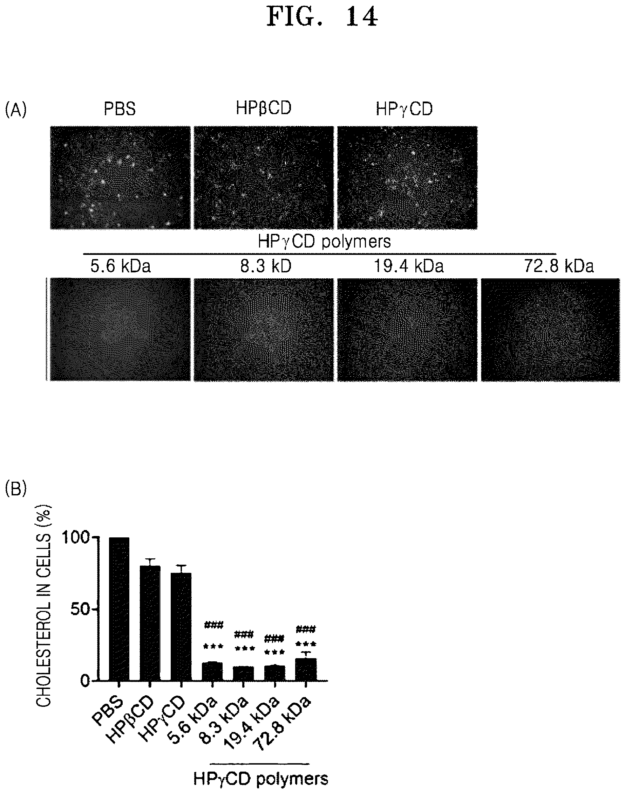

FIG. 14 shows, in FIG. 14A, representative images of cholesterol remaining in the cells after macrophages that had taken up fluorescent cholesterol were treated with hydroxypropyl-gamma-cyclodextrin polymer in various sizes, and in FIG. 14B, the quantified cholesterol amounts. ***P < 0.001 is a value obtained by comparing with hydroxypropyl-beta-cyclodextrin, ###P < 0.001 is a value obtained by comparing with hydroxypropyl-gamma-cyclodextrin, and One-way ANOVA analysis and Tukey's post-hoc analysis were used. Data are expressed as mean ± SD (where n = 3);

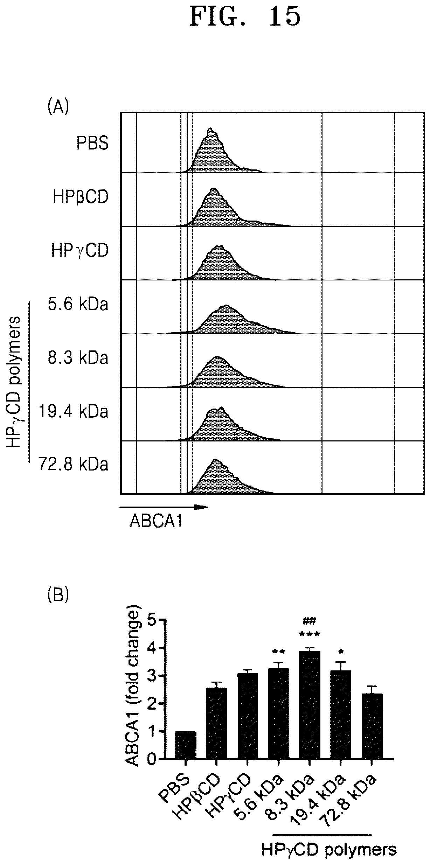

FIG. 15 shows the level of ABCA1 expressed in the cells after macrophages that had taken up fluorescent cholesterol were treated with hydroxypropyl-gamma-cyclodextrin polymer in various sizes, (FIG. 15A) and the the quantified amounts of ABCA1 (FIG. 15B). *P < 0.05, **P < 0.01, and ***P < 0.001 are values obtained by comparing with hydroxypropyl-beta-cyclodextrin, ##P < 0.01 is a value obtained by comparing with hydroxypropyl-gamma-cyclodextrin, and One-way ANOVA analysis and Tukey's post-hoc analysis were used. Data are expressed as mean ± SD (where n = 3);

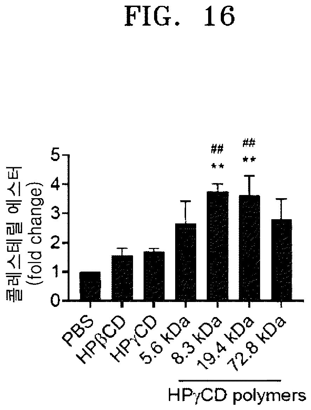

FIG. 16 shows the level of intracellular cholesteryl ester after macrophages that had taken up fluorescent cholesterol were treated with hydroxypropyl-gamma-cyclodextrin polymer in various sizes. **P < 0.01 is a value obtained by comparing with hydroxypropyl-beta-cyclodextrin, ##P < 0.01 is a value obtained by comparing with the hydroxypropyl-gamma-cyclodextrin, and One-way ANOVA analysis and Tukey's post-hoc analysis were used. Data are expressed as mean ± SD (where n = 3);

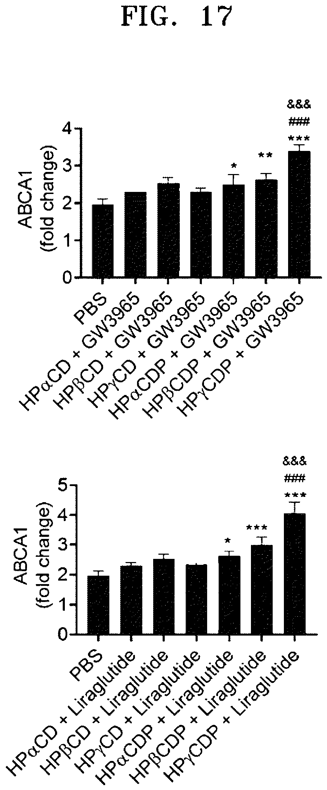

FIG. 17 shows the level of ABCA1 expressed in the cells after macrophages that had taken up fluorescent cholesterol were treated with cyclodextrin and GW3965 in combination (top) or cyclodextrin and liraglutide in combination (bottom), respectively. *P < 0.05, **P < 0.01, and ***P < 0.001 are values obtained by comparing with PBS, ###P < 0.001 is a value obtained by comparing with hydroxypropyl-beta-cyclodextrin polymer + GW3965 or liraglutide, &&&P < 0.001 is a value obtained by comparing with hydroxypropyl-alpha-cyclodextrin polymer + GW3965 or liraglutide, and One-way ANOVA analysis and Tukey's post-hoc analysis were used. Data are expressed as mean ± SD (where n = 3);

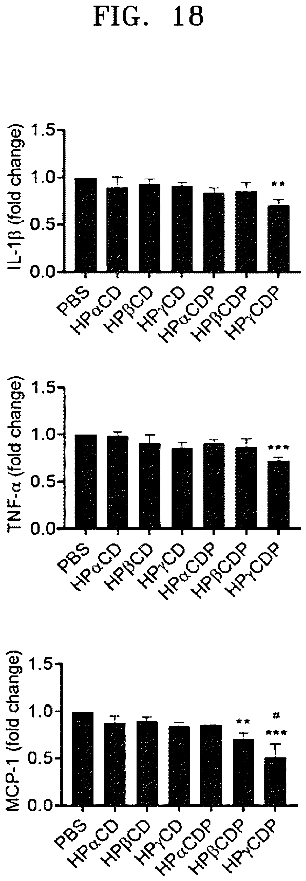

FIG. 18 shows the levels of inflammatory cytokines (IL-1β: top, MCP-1: middle, and TNF-α: bottom) secreted from the cells after macrophages had taken up cholesterol to cause an inflammatory response and then were treated with cyclodextrin. **P < 0.01 and ***P < 0.001 are values obtained by comparing with PBS, #P < 0.05 is a value obtained by comparing with hydroxypropyl-beta-cyclodextrin polymer, and One-way ANOVA analysis and Tukey's post-hoc analysis were used. Data are expressed as mean ± SD (where n = 3);

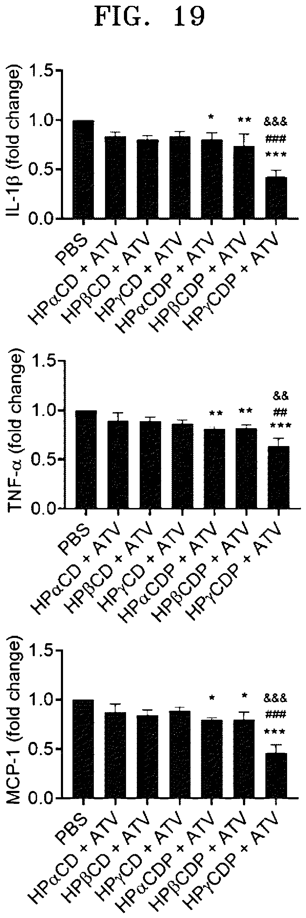

FIG. 19 shows the levels of inflammatory cytokines (IL-1β: top, MCP-1: middle, and TNF-α: bottom) secreted from the cells after macrophages had taken up cholesterol to cause an inflammatory response and then were treated with cyclodextrin and atorvastatin in combination. *P < 0.05, **P < 0.01, and ***P < 0.001 are values obtained by comparing with PBS, ##P < 0.01 and ###P < 0.001 are values obtained by comparing with hydroxypropyl-beta-cyclodextrin polymer + atorvastatin, &&P < 0.01 and &&&P < 0.001 are values obtained by comparing with hydroxypropyl-alpha-cyclodextrin polymer + atorvastatin, and One-way ANOVA analysis and Tukey's post-hoc analysis were used. Data are expressed as mean ± SD (where n = 3). ATV represents atorvastatin;

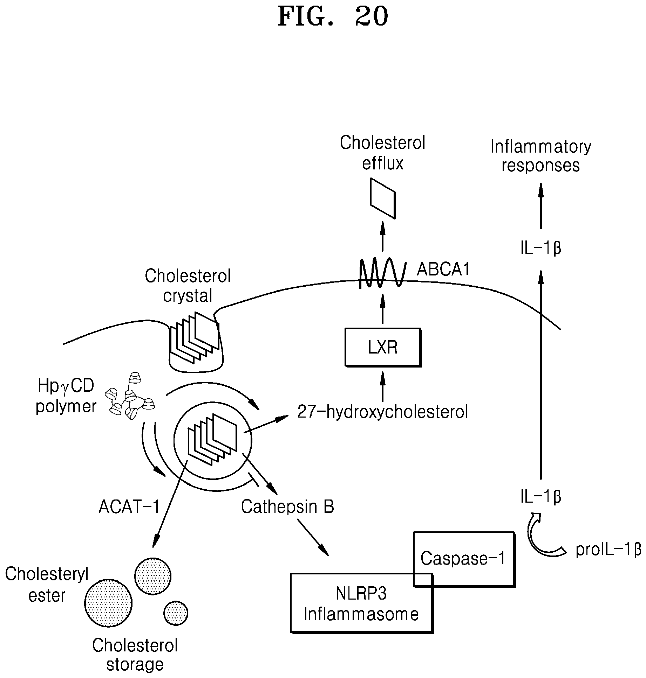

FIG. 20 is a schematic view illustrating the effect of hydroxypropyl-gamma-cyclodextrin on intracellular cholesterol metabolism and excretion and =anti-inflammatory responses. ;

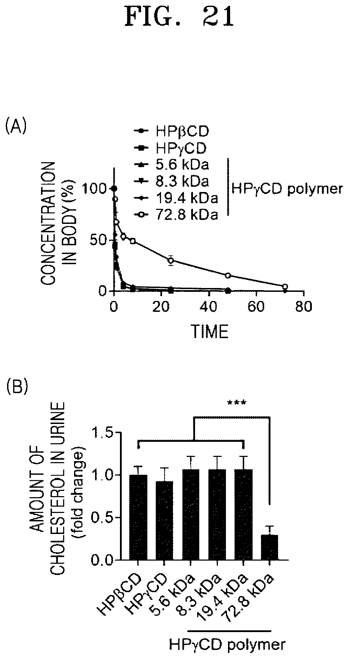

FIG. 21 shows the *in vivo* half-life of cholesterol in blood after intravenously injecting 500 mg/kg of a cyclodextrin-cholesterol inclusion complex based on cyclodextrin into C57BL/6 mice (FIG. 21A) and the amounts of cholesterol excreted in urine (FIG. 21B). ***P < 0.001, and One-way ANOVA analysis and Tukey's post-hoc analysis were used. Data are expressed as mean ± SD (where n = 3);

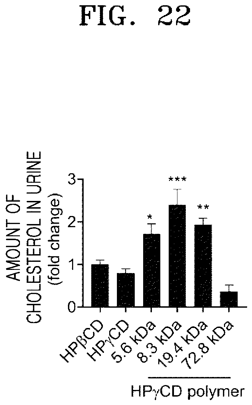

FIG. 22 shows the amounts of cholesterol excreted in urine after subcutaneously injecting 4 g/kg of cyclodextrin into C57BU6 mice. *P < 0.05, **P < 0.01, and ***P < 0.001 are values obtained by comparing with hydroxypropyl-beta-cyclodextrin, and One-way ANOVA analysis and Tukey's post-hoc analysis were used. Data are expressed as mean ± SD (where n = 3);

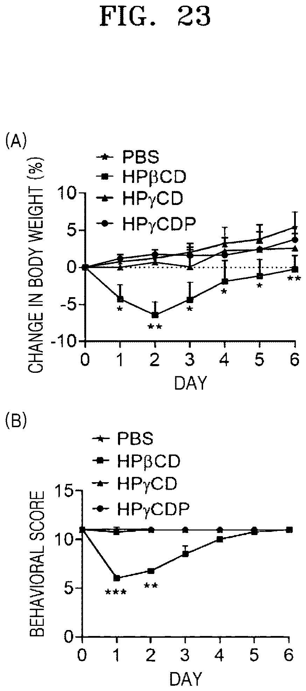

FIG. 23 shows the changes in the body weight (FIG. 23A) and behavioral score (FIG. 23B) a week after subcutaneously injecting 10 g/kg of cyclodextrin into C57BL/6 mice. *P < 0.05, **P < 0.01, and ***P < 0.001 are values obtained by comparing with PBS, and Two-way Repeated Measures ANOVA analysis and Tukey's post-hoc analysis were used. Data are expressed as mean ± SD (where n = 5);

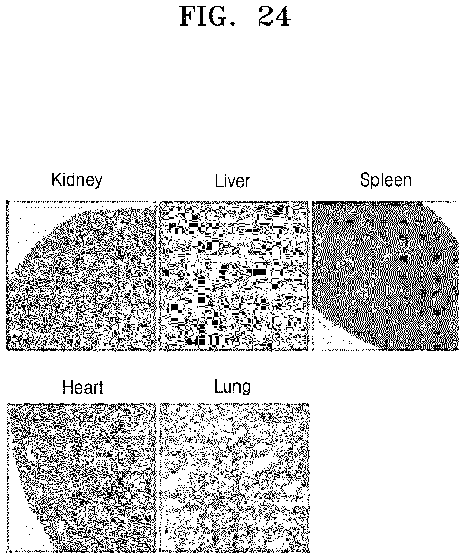

FIG. 24 shows representative histology of major organs a week after subcutaneously injecting 10 g/kg of cyclodextrin into C57BL/6 mice; and

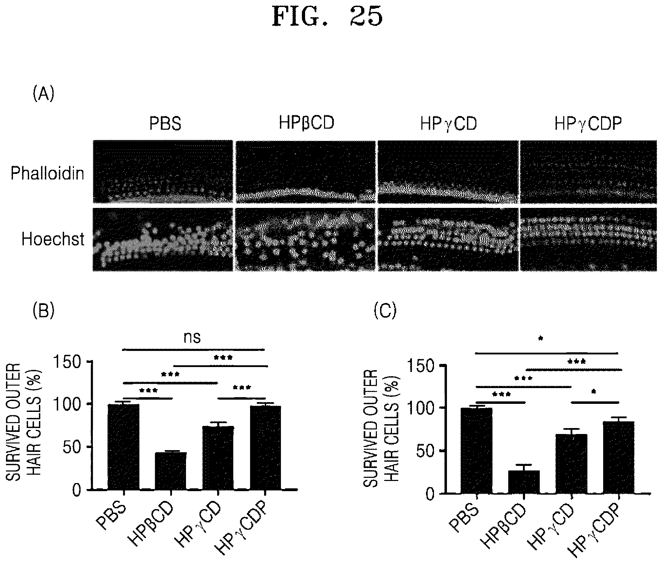

FIG. 25 showsthe ototoxicity a week after subcutaneously injecting 10 g/kg of cyclodextrin and intraventricularly injecting 5 g/kg brain weight of cyclodextrin into C57BL/6 mice, wherein FIG. 25A shows the results of observing the outer hair cells through confocal microscopy one week after subcutaneous injection, FIG. 25B shows quantification of the outer hair cells that survived one week after subcutaneous injection, and FIG. 25C shows quantification of the outer hair cells that survived one week after intraventricular injection. *P < 0.05 and ***P < 0.001, and One-way ANOVA analysis and Tukey's post-hoc analysis were used. Data are expressed as mean ± SD (where n = 3).

### MODE OF DISCLOSURE

Hereinafter, an aspect of an embodiment will be described in detail according to examples. However, these examples are provided to illustratively describe an aspect of an embodiment, and the scope of an embodiment is not limited to these examples. The examples of an embodiment may provide more complete description of an embodiment to those of ordinary skill in the art.

### Example 1. Preparation of cyclodextrin polymer

Hydroxypropyl-gamma-cyclodextrin was dissolved in 500 mL of a NaOH aqueous solution (33 % w/w) in concentrations of 500 mg/mL, 350 mg/mL, 250 mg/mL, and 125 mg/mL, and sufficiently stirred for 24 hours at room temperature to induce alkoxide groups. To cross-link the alkoxide groups, epichlorohydrin was added at ratios of 0.5, 1, 3, 5, 7, and 10 mol per 1 mol of cyclodextrin, and the resultants were stirred for 48 hours at room temperature. To cease the polymerization, 500 mL of acetone was added thereto, and the resultants were remained at room temperature for 30 minutes. After removing acetone in the supernatant by decantation, the resultants were remained in an oven at 50 °C for 24 hours. 10 N HCl was used to adjust the pH of the solutions to 12. To obtain the finally synthesized cyclodextrin polymer, dialysis was performed using a dialysis membrane and distilled water for 72 hours, and the distilled water was replaced twice a day. After passing the collected mixture through a 0.45 µm polyvinylidene fluoride (PVDF) filter, the resultant was lyophilized to obtain a powder and stored at room temperature. The occurrence of gelation and time for gelation depending on the concentration of cyclodextrin and the molar ratio of epichlorohydrin to hydroxypropyl-gamma-cyclodextrin is shown in Table 2.

**[Table 2]**

| | | **Molar ratio (Epichlorohydrin/cyclodextrin)** | | | | | |
|---|---|---|---|---|---|---|---|
| | | **0.5** | **1** | **3** | **S** | **7** | **10** |
| **Cyclodextrin concentration (mg/ml)** | **500** | - | - | - | **O (<24 hours)** | **O (<8hours)** | **O (<1 hour)** |
| | **350** | - | - | - | - | **O (<24 hours)** | **O (<8 hours)** |
| | **250** | - | - | - | - | - | - |
| | **125** | - | - | - | - | - | - |

As shown in Table 2, when the concentration of cyclodextrin was 350 mg/mL or more, gelation of the cyclodextrin solution was observed within 48 hours as the molar ratio of epichlorohydrin to hydroxypropyl-gamma-cyclodextrin increased, and this confirmed the formation of non-water-soluble polymer. Also, when the concentration of cyclodextrin was 250 mg/mL or less, gelation of the solution was not observed even at the highest molar ratio of 10, showing that the formation of a water-soluble polymer may be easily induced.

### Example 2. Observation of molecular weight, size, and shape of cyclodextrin polymer

Zetasizer Nano ZS90 (Malvern Instruments, Malvern, UK) was used to analyze the molecular weights and sizes of the cyclodextrin polymers. Static light scattering was used for the molecular weight measurement, the scattering intensity (Kc/Rθ) of various cyclodextrin concentrations was measured at a single angle, and toluene was used as a reference sample. The molecular weights were calculated using Debye-plot, and the molecular weights were obtained in values in Kc/Rθ when the concentration was 0. Also, hydrodynamic diameters of the cyclodextrin polymers were measured in the cyclodextrin solution (5 mg/mL) using dynamic light scattering. Moreover, shapes of the cyclodextrin polymers were observed using a scanning electron microscope. The results showing the molecular weights (kDa) of the polymers formed in Example 1 are shown in Table 3, the hydrodynamic diameters of the polymers formed under the condition of the cyclodextrin concentration of 250 mg/mL are shown in FIG. 1A, and the representative size distribution of the polymers when the molar ratios are 0.5, 1, 3, and 10 are shown in FIG. 1B. Also, the scanning electron microscopic images of the hydroxypropyl-gamma-cyclodextrins prepared at the molar ratios are shown in FIG. 2.

**[Table 3]**

| | | **Molar ratio Epichlorohydrin/cyclodextrin** | | | | | |
|---|---|---|---|---|---|---|---|
| | | **0.5** | **1** | **3** | **5** | **7** | **10** |
| **Cyclodextrin concentration (mg/ml)** | **500** | **23 ± 9.0** | **89 ± 32.5** | **353 ± 53.4** | - | - | - |
| | **350** | **13.3 ± 9.5** | **28.3 ± 12** | **63.4 ± 22** | **158 ± 45** | - | - |
| | **250** | **5.6 ± 0.32** | **8.3 ± 0.47** | **19.4 ± 3.6** | **25.3 ± 5.2** | **52.0 ± 3.9** | **72.8 ± 11.5** |
| | **125** | **4.8 ± 0.25** | **5.6 ± 0.55** | **6.31 ± 1.4** | **5.8 ± 3.25** | **8.3 ± 3.5** | **11.3 ±** 3.5 |

As shown in Table 3, when the reaction concentration of cyclodextrin was 250 mg/mL, polymers in various sizes were induced using various molar ratios of epichlorohydrin to cyclodextrin. However, when the concentration of cyclodextrin was 350 mg/mL or more, polymers having a molecular weight of 60 kDa or more, of which glomerular filtration is not easy, were mainly formed, and uneven particle distribution was confirmed. Also, when the concentration of cyclodextrin was 125 mg/mL or less, polymers in various sizes were not obtained with the increase in the molar ratio, showing that effective polymerization did not occur.

As shown in FIG. 1A, when the reaction concentration was 250 mg/mL, hydroxypropyl-gamma-cyclodextrin polymers having hydrodynamic diameters of 4 ± 0.3 nm, 5.7 ± 0.25 nm, 6.0 ± 0.6 nm, 8.5 ± 1.7 nm, 8.7 ± 0.85 nm, and 10.7 ± 0.7 nm were obtained after polymerization using the molar ratios of 0.5, 1, 3, 5, 7, and 10, respectively. Also, in FIG. 1B, the polydispersity index (PDI) showed that the resultants obtained using each molar ratio formed polymers having even distribution. As shown in FIG. 2, hydroxypropyl-gamma-cyclodextrin polymers in different sizes depending on the molar ratios of epichlorohydrin were effectively formed as observed by scanning electron microscopy. In addition, in FIG. 3, the formation of hydroxypropyl-gamma-cyclodextrin polymers in different sizes was confirmed by gel permeation chromatography. The results of analyzing the hydroxypropyl-gamma-cyclodextrin polymers using a Bruker AVANCE II 500MHz NMR are shown in FIG. 4. Therefore, it was concluded that water-soluble hydroxypropyl-gamma-cyclodextrin polymers having different sizes may be effectively prepared using the polymerization conditions.

### Example 3. Assessment of plasma membrane cholesterol extraction

Raw 264.7 cells were maintained in a Dulbecco's modified Eagle's medium (DMEM) containing 10 % fetal bovine serum (FBS) and 1 % penicillin/streptomycin. In order to suppress endocytosis of cyclodextrin and induce only plasma membrane cholesterol extraction, 1 × 10⁵ cells were fixed at room temperature for 20 minutes using formaldehyde, and then cyclodextrin was dissolved in PBS in a concentration of 20 mg/mL and treated to the cells at 37 °C for 1 hour. The treated PBS was collected, centrifuged at a rate of 14,000 g for 30 minutes to remove cell debris and microvesicles, and then the cholesterol concentration in the supernatant was quantified using a Cholesterol Quantification Kit (Sigma Aldrich). The plasma membrane cholesterol extraction of gamma-cyclodextrins and polymers thereof was compared with alpha and beta-cyclodextrins and polymers thereof. The hydroxypropyl-alpha-cyclodextrin and hydroxypropyl-beta-cyclodextrin polymers were polymers prepared in the same manner as in the hydroxypropyl-gamma-cyclodextrin under the condition of 250 mg/mL of the concentration. The plasma membrane cholesterol extraction induced by gamma-cyclodextrins and polymers thereof was compared with alpha- and beta-cyclodextrins and polymers thereof, and the results are shown in FIG. 5.

As shown in FIG. 5, the hydroxypropyl-gamma-cyclodextrin monomers and polymers showed significantly low levels of cholesterol extraction as compared to those of the hydroxypropyl-alpha-cyclodextrin and hydroxypropyl-beta-cyclodextrin monomers and polymers across all the molecular weights examined. In the case of the monomers, the larger amount of plasma membrane cholesterol extraction was induced in the order of hydroxypropyl-beta-cyclodextrin (45 %), hydroxypropyl-alpha-cyclodextrin (42.8 %), and hydroxypropyl-gamma-cyclodextrin (5 %). From these results, it was confirmed that hydroxypropyl-gamma-cyclodextrin monomers exhibit physicochemical properties that do not induce plasma membrane cholesterol extraction.

In the case of the polymers, it was confirmed that hydroxypropyl-alpha-cyclodextrin, hydroxypropyl-beta-cyclodextrin, and hydroxypropyl-gamma-cyclodextrin induced at maximum 77.8 %, 93.9 %, and 6.7 % of plasma membrane cholesterol extraction, respectively. The polymers having a molecular weight of about 80 kDa tended to extract plasma membrane cholesterol less than those of the monomers. A tendency in the plasma membrane cholesterol removal efficiency, which increases and then decreases with the increase in the size of the polymers of cyclodextrin was observed. Overall, it was shown that the physicochemical properties of the monomers and the polymer structures (could contribute to the plasma membrane cholesterol removal efficiency )and the hydroxypropyl-gamma-cyclodextrin polymers exhibited significantly low plasma membrane cholesterol removal efficiency as compared to those of other cyclodextrin polymers.

### Example 4. Assessment of cytotoxicity

Raw 264.7 cells were maintained in a DMEM containing 10 % FBS and 1 % penicillin/streptomycin. 5 × 10⁴ cells were dispensed in each well of a 96-well plate, and after 24 hours, the cells were treated with cyclodextrin at a concentration of 20 mg/mL. After culturing the cells at 37 °C in 5 % CO₂, a CCK-8 agent was added, 10 µL at a time. After 3 hours, absorbance was measured at 450 nm to assess the cell viability with the hydroxypropyl-alpha-, -beta-, and -gamma-cyclodextrin monomers and their polymers with varying molecular weights, as shown in FIG. 6.

As shown in FIG. 6, the hydroxypropyl-gamma-cyclodextrin monomers and polymers had significantly low cytotoxicity as compared to those of the alpha- and beta-cyclodextrins regardless of their molecular weights. In the case of the monomers, the lower cell viability was observed in the order of hydroxypropyl-beta-cyclodextrin (9.6 %), hydroxypropyl-alpha-cyclodextrin (14.2 %), and hydroxypropyl-gamma-cyclodextrin (93.9 %). Cytotoxicity by cyclodextrin is known to be caused by excessive extraction of plasma membrane cholesterol, and correspondingly, a positive correlation between the degree of cholesterol extraction and cytotoxicity was confirmed. Hydroxypropyl-alpha-cyclodextrin and hydroxypropyl-beta-cyclodextrin polymers had increased cell viability at the molecular weight of about 80 kDa than that of each of the monomers thereof, but had significantly high cytotoxicity as compared to that of the hydroxypropyl-gamma-cyclodextrin. Therefore, it was confirmed that although the structure and size of the polymer influence plasma membrane cholesterol extraction, the type of the monomer may be more important, and that cytotoxicity may be minimized using a hydroxypropyl-gamma-cyclodextrin-based polymer.

### Example 5. Assessment of hemolytic activity

For hemolytic activity analysis, 30 µL of whole blood from wild-type C57/BL6 mice was mixed with 300 µL of a PBS solution containing 50 mg/mL of cyclodextrin and reacted at 37 °C for 1 hour. After centrifuging the mixture at 3,000 g for 5 minutes, the absorbance at 540 nm was measured using the supernatant. As a positive control, an ammoniumchloride-potassium (ACK) lysis buffer used for red blood cell lysis was used, and hemolytic activities depending on the molecular weight of hydroxypropyl-alpha-, -beta-, and -gamma-cyclodextrin monomers and polymers were confirmed, and the results are shown in FIG. 7.

As shown in FIG. 7, the hydroxypropyl-gamma-cyclodextrin monomers and polymers had significantly low hemolytic activities as compared to those of the alpha- and beta-cyclodextrins regardless of their molecular weights. In the case of the monomers, the higher hemolytic activity was observed in the order of hydroxypropyl-beta-cyclodextrin (97.1 %), hydroxypropyl-alpha-cyclodextrin (93.5 %), and hydroxypropyl-gamma-cyclodextrin (1.6 %). As the molecular weights of the hydroxypropyl-alpha-cyclodextrin and hydroxypropyl-beta-cyclodextrin increased, the hemolytic activities decreased. In addition, hemolysis by cyclodextrin is also caused by excessive extraction of plasma membrane cholesterol, and accordingly, a positive correlation between the degree of cholesterol extraction and hemolytic activity was confirmed. Hydroxypropyl-alpha-cyclodextrin and hydroxypropyl-beta-cyclodextrin polymers had decreased hemolytic activity at the molecular weight of about 80 kDa than that of each of the monomers thereof, but had significantly high hemolytic activity as compared to that of the hydroxypropyl-gamma-cyclodextrin. Therefore, it was confirmed that although the structure and size of the polymer influence plasma membrane cholesterol extraction, the type of the monomer may be more important, and that hemolytic activity may be minimized using a hydroxypropyl-gamma-cyclodextrin-based polymer.

### Example 6. Assessment of cholesterol crystal dissolution

First, in order to form cholesterol crystals, a solution including 2 mg/ml of cholesterol and 0.2 mg/ml of NBD-cholesterol in ethanol was prepared, and then distilled water (30 % v/v) was added to the solution to induce crystallization. The solution was completely dried under vacuum, resuspended in PBS, and sonicated for 30 seconds. 20 µL of 5 mM cholesterol was mixed with 300 µL of 20 mg/mL cyclodextrin and reacted at 37 °C for 2 hours. After centrifuging the mixture at 14,000 g for 30 minutes to remove undissolved cholesterol crystals, the supernatant was collected and the fluorescence of dissolved NBD-cholesterol was measured at 480 nm/520 nm, and the cholesterol crystal dissolution levels depending on the molecular weights of hydroxypropyl-alpha-, -beta-, - gamma-cyclodextrin monomers and polymers were confirmed. The results are shown in FIG. 8.

As shown in FIG. 8, in the case of the monomers, the higher cholesterol crystal dissolution was observed in the order of hydroxypropyl-beta-cyclodextrin (32.3 %), hydroxypropyl-alpha-cyclodextrin (21.9 %), and hydroxypropyl-gamma-cyclodextrin (12.1 %). In the case of the polymers, it was confirmed that cholesterol-solubilizing ability increased as the molecular weight increased, and that the hydroxypropyl-gamma-cyclodextrin polymer had the most increase in efficiency. The heat map based on the data on cell membrane cholesterol extraction, cytotoxicity, and hemolytic activity of the cyclodextrin monomers and 3 kDa to 30 kDa polymers is shown in FIG. 9, and through this, it was concluded that the hydroxypropyl-gamma-cyclodextrin polymer has low cytotoxicity and low hemolytic activity caused by plasma membrane cholesterol extraction and effectively induces cholesterol dissolution.

Overall, as described in the schematic view of FIG. 10, due to the physiochemical properties of hydroxypropyl-gamma-cyclodextrin and its polymer structure, the hydroxypropyl-gamma-cyclodextrin polymer has low reactivity with respect to plasma membrane cholesterol and effectively reacts with and dissolves cholesterol crystals.

### Example 7. Assessment of intracellular cholesterol excretion and cholesterol metabolism of cyclodextrin polymer per condition

### 7.1 Assessment of intracellular cholesterol excretion and cholesterol metabolism of cyclodextrin polymer

Raw 264.7 cells were dispensed at 5 × 10⁴ cells per well of a 96-well plate, and after 24 hours, the cells were treated with cholesterol crystals at a concentration of 50 µM. After 3 hours, the cholesterol crystals not ingested into the cells were removed through medium replacement and then treated with cyclodextrin monomers and polymers of 3 kDa to 30 kDa at a concentration of 5 mg/mL. The cells were cultured at 37 °C in 5 % CO₂ for 48 hours, and intracellular cholesterol fluorescence was analyzed using a fluorescence microscope. In addition, to investigate the effect of cyclodextrin on intracellular cholesterol metabolism, the amount of ATP-binding cassette transporter (ABCA1) involved in cholesterol excretion was quantified using a cell flow rate analyzer, and cholesteryl ester, which is a storage form of cholesterol, was quantified. The results are shown in FIG. 11.

As shown in FIG. 11, among cyclodextrins and the cyclodextrin polymers, the hydroxypropyl-gamma-cyclodextrin polymer most significantly increased intracellular cholesterol excretion, and induced an increase in ABCA1 and cholesteryl ester levels.

### 7.2 Assessment of intracellular cholesterol excretion and cell viability depending on the concentration of cyclodextrin polymer

An experiment was performed to confirm intracellular cholesterol excretion and cell viability depending on the concentration of the cyclodextrin polymer. Intracellular cholesterol excretion and cellular activity of cells when the cells were treated with cyclodextrin polymers of various concentrations of 0.1 mg/mL, 1 mg/mL, 5 mg/mL, 10 mg/mL, 15 mg/mL, and 20 mg/mL through a concentration gradient were assessed, and the results are shown in FIG. 12. The experiment conditions were the same as in Example 7.1, except that the concentrations were changed.

As shown in FIG. 12, the hydroxypropyl-alpha and -beta-cyclodextrin polymers exhibited concentration-dependent cholesterol excretion, but as the concentration increased, a decrease in the cell viability occurred along with cytotoxicity. On the other hand, in the case of the hydroxypropyl-gamma-cyclodextrin, concentration-dependent cholesterol excretion effect was obtained without cytotoxicity even at a high concentration. These results show that the hydroxypropyl-gamma-cyclodextrin polymers not only induce significantly high cholesterol excretion as compared to those of other polymers at the same concentration, but also do not generate cytotoxicity even at a high concentration, enabling high-dose treatment.

### 7.2 Assessment of intracellular cholesterol excretion and cell viability depending on substituent of cyclodextrin polymer

An experiment was performed to confirm whether intracellular cholesterol excretion and cell viability may change depending on the substituent of the cyclodextrin polymer. In particular, the alpha-cyclodextrin polymer, hydroxypropyl-alpha-cyclodextrin polymer, sulfobutylether-alpha-cyclodextrin polymer, beta-cyclodextrin polymer, hydroxypropyl-beta-cyclodextrin polymer, sulfobutylether-beta-cyclodextrin polymer, gamma-cyclodextrin polymer, hydroxypropyl-gamma-cyclodextrin polymer, and sulfobutylether-gamma-cyclodextrin polymer of 3 kDa to 30 kDa at a concentration of 20 mg/mL were used to confirm cytotoxicity and cholesterol excretion ability, and the results are shown in FIG. 13. The experiment conditions were the same as in Example 7.1, except that the concentrations and the substituents of the cyclodextrin polymer were changed.

As shown in FIG. 13, it was confirmed that the toxicity was low and effective in cholesterol excretion in the order of hydroxypropyl-gamma-cyclodextrin, sulfobutylether-gamma-cyclodextrin, and gamma-cyclodextrin polymer. Therefore, it was confirmed that the gamma-cyclodextrin-based polymer is useful in lowering cytotoxicity and improving cholesterol metabolism. Particularly, when the substituent of the polymer is a hydroxypropyl, the polymer had the lowest cytotoxicity and excellent cholesterol excretion ability.

### 7.4 Assessment of intracellular cholesterol excretion efficiency and cholesterol processing metabolism depending on the molecular weight of cyclodextrin polymer

An experiment was performed to confirm whether intracellular cholesterol excretion may change depending on the molecular weight of the cyclodextrin polymer. The cells were treated with hydroxypropyl-beta-cyclodextrin, hydroxypropyl-gamma-cyclodextrin, and hydroxypropyl-gamma-cyclodextrin polymers of 5.6 kDa, 8.3 kDa, 19.4 kDa, and 72.8 kDa at a concentration of 5 mg/mL. All the experiment conditions were the same as in Example 7.1, except these conditions, and cells were treated with the hydroxypropyl-gamma-cyclodextrin polymers of various sizes (molecular weights of the polymers) and the amounts of cholesterol remaining in the cells were imaged, as shown in FIG. 14A, and the quantified results are shown in FIG. 14B.

As shown in FIGS. 14A and 14B, an intracellular cholesterol level of PBS, which is the control, was set to be 100 %, meaning that no cholesterol excretion occurred at all. In comparison, in the groups treated with hydroxypropyl-beta-cyclodextrin and hydroxypropyl-gamma-cyclodextrin, the amounts of intracellular cholesterol were 80.5% and 75%, respectively. Also, in the groups treated with the hydroxypropyl-gamma-cyclodextrin polymers of 5.6 kDa, 8.3 kDa, 19.4 kDa, and 72.8 kDa, the amounts of intracellular cholesterol were 12.8 %, 10 %, 11 %, and 16 %, respectively. The polymer groups exhibited on average about 5 times higher cholesterol excretion efficiency than those of the monomers.

Also, an experiment was performed to assess cholesterol metabolism based on the expression of ABCA1. In this experiment, the cells were treated with hydroxypropyl-beta-cyclodextrin, hydroxypropyl-gamma-cyclodextrin, and hydroxypropyl-gamma-cyclodextrin polymers of 5.6 kDa, 8.3 kDa, 19.4 kDa, and 72.8 kDa at a concentration of 5 mg/mL. All the experiment conditions were the same as in Example 7.1, except these conditions, and amounts of ABCA1 expressed in the cells are shown in representative histograms, as shown in FIG. 15A, and the quantified results are shown in FIG. 15B.

As shown in FIGS. 15A and 15B, the hydroxypropyl-gamma-cyclodextrin polymers having various molecular weights remarkably increased the ABCA1 expression of the cells as compared with those of the control, PBS, and the monomers. The groups treated with the hydroxypropyl-gamma-cyclodextrin polymers of 5.6 kDa, 8.3 kDa, 19.4 kDa, and 72.8 kDa exhibited ABCA1 expression increased by 3.3, 3.9, 3.2, and 2.4 times, respectively, as compared to those of the control group, PBS.

Also, cholesteryl ester levels in the cells after macrophages that had taken up fluorescent cholesterol were treated with the hydroxypropyl-gamma-cyclodextrin polymer in various sizes were assessed. The cells were treated with of hydroxypropyl-beta-cyclodextrin, hydroxypropyl-gamma-cyclodextrin, and hydroxypropyl-gamma-cyclodextrin polymers of 5.6 kDa, 8.3 kDa, 19.4 kDa, and 72.8 kDa at a concentration of 5 mg/mL. All the experiment conditions were the same as in Example 7.1, except these conditions, and the results are shown in FIG. 16.

As shown in FIG. 16, the hydroxypropyl-gamma-cyclodextrin polymers having various sizes remarkably increased the amounts of cholesteryl ester as compared with those of PBS and the monomers. It was confirmed that the groups treated with the hydroxypropyl-gamma-cyclodextrin polymers of 5.6 kDa, 8.3 kDa, 19.4 kDa, and 72.8 kDa exhibited the amounts of cholesteryl ester increased by 2.7, 3.8, 3.6, and 2.8 times, respectively, as compared to those of PBS(control group).

Overall, the hydroxypropyl-gamma-cyclodextrin polymer significantly improved intercellular cholesterol metabolism as compared to those of the hydroxypropyl-alpha-, and -beta-cyclodextrin polymers. Also, such effect was observed in a range of molecular weights.

### Example 8. Assessment of the effect of combination of cyclodextrin polymer and other drugs

An experiment was conducted to determine whether a synergistic effect may occur since cholesterol metabolism may further be improved when the cyclodextrin polymer is administered in combination with other drugs, other than the single administration of the cyclodextrin polymer.

In particular, hydroxypropyl-alpha-cyclodextrin, hydroxypropyl-beta-cyclodextrin, and hydroxypropyl-gamma-cyclodextrin monomers and hydroxypropyl-alpha-cyclodextrin, hydroxypropyl-beta-cyclodextrin, and hydroxypropyl-gamma-cyclodextrin polymers at 2 mg/mL of each; and GW3965, which is an agonist of the liver X receptor (LXR) that plays an important role in cholesterol regulation, and Liraglutide, which is an agonist of GLP-1, at 1 µM and 100 nM, respectively, were in combination with the cyclodextrin monomers and polymers to macrophages ingested with cholesterol. Thereafter, the intracellular ABCA1 expression levels were quantified in comparison with the control, PBS, and the results of co-administration with GW3965 and the results of co-administration with liraglutide are shown in FIGS. 17A and 17B, respectively.

As shown in FIGS. 17A and 17B, GW3965 and liraglutide had a synergistic effect by inducing a significant increase in the amount of ABCA1 when treated in combination with a hydroxypropyl-gamma-cyclodextrin polymer.

These results show that the hydroxypropyl-gamma-cyclodextrin polymer can significantly improve cholesterol metabolism in combination with other drugs compared to other monomers or polymers.

### Example 9. Assessment of anti-inflammatory effect of cyclodextrin polymer

### 9.1 Assessment of anti-inflammatory effect through treatment of cyclodextrin polymer

Cholesterol crystals ingested into cells are known to induce the activation of NLRP3 inflammasome and to cause various inflammatory responses. Thus, an experiment was performed to determine whether anti-inflammatory activity may appear in the case of treating with the alpha, beta or gamma cyclodextrin monomer(s and polymers )of the above example.

Raw 264.7 cells were dispensed at 5 × 10⁴ cells per well of a 96-well plate, and after 24 hours, the cells were treated with cholesterol crystals at a concentration of 50 µM. After 3 hours, the cholesterol crystals not ingested into the cells were removed through medium replacement and then treated with cyclodextrin monomers and polymers of 3 kDa to 30 kDa at a concentration of 5 mg/mL. After culturing the cells at 37 °C in 5 % CO₂, the inflammatory cytokines, that are interleukin-1β (IL-1β), monocyte chemoattractant protein-1 (MCP-1), and tumor necrosis factor-α (TNF-α) in the culture medium were quantified using an enzyme-linked immunosorbent assay (ELISA), and the results of quantifying the amounts of inflammatory cytokines secreted from the cells are shown in FIG. 18.

As shown in FIG. 18, at a concentration of 5 mg/mL, the hydroxypropyl-gamma-cyclodextrin polymer significantly inhibited the secretion of IL-1β, MCP-1, and TNF-α compared to the control treated with PBS alone. The hydroxypropyl-beta-cyclodextrin polymer significantly inhibited the secretion of MCP-1 compared to the control group, but did not significantly inhibit other inflammatory cytokines. It was confirmed that the cyclodextrin monomers did not show significant cytokine inhibition.

### 9.1 Assessment of anti-inflammatory effect through combination of cyclodextrin polymer with other drugs

An experiment was conducted to determine whether a synergistic effect on the anti-inflammatory effect may occur when the cyclodextrin polymer and atorvastatin (ATV), which has an anti-inflammatory effect, are co-administered.

Hydroxypropyl-alpha-cyclodextrin, hydroxypropyl-beta-cyclodextrin, and hydroxypropyl-gamma-cyclodextrin monomers and hydroxypropyl-alpha-cyclodextrin, hydroxypropyl-beta-cyclodextrin, and hydroxypropyl-gamma-cyclodextrin polymers at a concentration of 2 mg/mL of each; and atorvastatin at a concentration of 2 µM were co-administered to macrophages ingested with cholesterol. Conditions other than co-administration were carried out in the same manner as in Example 9.1, and changes in the secreted amounts of inflammatory cytokines of IL-1β, TNF-α and MCP-1 were quantified and shown in FIG. 19.

As shown in FIG. 19, the case of co-administration with the hydroxypropyl-gamma-cyclodextrin polymer most significantly inhibited inflammation, and thus a synergistic effect in terms of an anti-inflammation effect was confirmed.

Therefore, it was confirmed that the hydroxypropyl-gamma-cyclodextrin polymer may exhibit superior anti-inflammatory effects over the other types of polymers under both single and co-administration conditions Overall, as depicted in the schematic view illustrating intracellular cholesterol metabolism and excretion promotion and an anti-inflammatory reaction due to the hydroxypropyl-gamma-cyclodextrin of FIG. 20, it was concluded that the hydroxypropyl-gamma-cyclodextrin polymer can significantly improve intracellular cholesterol metabolism and excretion compared to other cyclodextrin monomers and polymers, and effectively inhibit the inflammatory responses caused by cholesterol metabolism dysregulation.

### Example 10. Assessment of cholesterol excretion from the body mediated by cyclodextrin

In order to assess cholesterol excretion from the body mediated by hydroxypropyl-gamma-cyclodextrin, 50 mg of polymers of different molecular weights were first dissolved in PBS, and reacted with 1 mg of NBD (2-(4-nitro-2,1,3-benzoxadiazol-7- yl) aminoethyl)-labeled cholesterol to form a cyclodextrin-cholesterol inclusion complex. Then, a dose of 500 mg/kg of cyclodextrin was intravenously injected into C57BU6 mice. The dose equivalents to 10 mg for a mouse weighing 20 g. The amount of NBD cholesterol in plasma was quantified at 3 minutes, 30 minutes, 1 hour, 4 hours, 8 hours, and 24 hours after injecting cyclodextrin through retro-orbital blood sampling, the urine was collected at 1 hour, 4 hours, and 24 hours to quantify the amount of NBD cholesterol, and the half-life of blood cholesterol in the body and the amount of urine excreted are shown in FIG. 21. Particularly, the results of the half-life of fluorescent cholesterol are shown in FIG. 21A, and the results of the amount of cholesterol in the excreted urine are shown in FIG. 21B.

As shown in FIG. 21A, the cyclodextrin monomers and the hydroxypropyl-gamma-cyclodextrin polymers having a molecular weight of 5.6 kDa, 8.3 kDa, 19.4 kDa, and 72.8 kDa showed the half-life in the blood of 0.9 hours, 1.1 hours, 1.3 hours, 2.0 hours, and 25 hours, respectively, meaning that the half-life in the blood increased with an increase in the size of the polymer. As shown in FIG. 21B, when the amount of NBD cholesterol in the urine was quantified at 1 hour, 4 hours, and 24 hours, it was confirmed that the cyclodextrin monomers and the hydroxypropyl-gamma-cyclodextrin polymers having a molecular weight of 5.6 kDa, 8.3 kDa, and 19.4 kDa showed similar amounts of cholesterol in the urine, but the polymer having a molecular weight of 72.8 kDa had the significantly decreased amount of cholesterol in the urine. From this, since the molecular weight fraction of glomerular filtration is about 60 kDa, it was confirmed that effective glomerular filtration did not occur for the polymers having a molecular weight greater than that. Therefore, it was confirmed that for the effective excretion of cholesterol from the body by the cyclodextrin-cholesterol inclusion complex, it is appropriate to use a polymer with a molecular weight less than the molecular weight cut-off for the glomerular filtration, but it is important to use a polymer showing critical significance because a polymer that is too small is rapidly excreted from the body.

Therefore, the polymers having a molecular weight of around 4 kDa to 60 kDa may be capable of effectively excreting cholesterol in the body by facilitating the excretion of cholesterol to the outside of the body through glomerular filtration while having an improved half-life.

In addition, to assess the ability of cyclodextrin to form inclusion complex withand mediate excretion of cholesterol in the body, the cyclodextrin polymer was administered (without pre-complexation with cholesterol) and the amount of excreted cholesterol in the urine was quantified.

4 g/kg of cyclodextrin was subcutaneously injected into C57BL/6 mice, and the urine was collected at 4 hours, 8 hours, and 24 hours, and the amounts of cholesterol in the urine were quantified using a Cholesterol Quantification Kit (Sigma Aldrich). The amounts of cholesterol excreted for 24 hours were all added up and compared, and the results are shown in FIG. 22.

As shown in FIG. 22, the amount of excretion of cholesterol from the body through urine was the highest when the hydroxypropyl-gamma-cyclodextrin polymers of 5.6 kDa, 8.3 kDa, and 19.4 kDa were used. Overall, it was shown that a hydroxypropyl-gamma-cyclodextrin polymer having an appropriate molecular weight may contribute to improving the formation of inclusion complex with cholesterol inside the body and its excretion.

### Example 11. Assessment of systemic toxicity and ototoxicity

To assess systemic toxicity, cyclodextrin dissolved in PBS was subcutaneously injected in an amount of 10 g/kg body weight to wild-type C57/BL6 mice. In the case of the polymers, hydroxypropyl-gamma-cyclodextrin polymers having a molecular weight of 3 kDa to 30 kDa were used. The body weight and behavioral characteristics of the mice were observed every day for a week. The behavioral characteristics of the mice were measured according to the criteria presented in Endpoints for Mouse Abdominal Tumor Models: Refinement of Current Criteria. In order to confirm toxicity to major organs, the mice were sacrificed 7 days later, and H&E staining was performed. In order to assess ototoxicity, the mice were sacrificed 7 days later, and the outer hair cells in the cochlea were quantified through microscopic observation. In addition, cyclodextrin dissolved in PBS was intraventricularly injected as much as 5,000 mg/kg brain weight using a 26 G syringe to confirm ototoxicity in direct delivery into the central nervous system. The brain weight of the mice was calculated to be about 0.4 g according to the age, and as a result, 2 mg of cyclodextrin was dissolved in 10 µL of PBS and injected. After 7 days, the outer hair cells in the cochlea were quantified through microscopic observation.

The weight change for one week are shown in FIG. 23A, and = the behavioral scores are shown in FIG. 23B. Also, the histological analysis of the major organs of the mice after one week is shown in FIG. 24, and ototoxicity of the C57BU6 mice was evaluated one week after subcutaneously injecting 10 g/kg of cyclodextrin and intraventricularly injecting 5 g/kg brain weight of cyclodextrin. The ototoxicity was observed with a confocal microscope. Particularly, the images of the outer hair cells one week after subcutaneous injection are shown in FIG. 25A, the quantified outer hair cells one week after the subcutaneous injection are shown in FIG. 25B, and the quantified outer hair cells one week after the intraventricular injection are shown in FIG. 25C.

As shown in FIG. 23A, in the case of hydroxypropyl-beta-cyclodextrin, weight loss was observed from the very next day after the 10 g/kg subcutaneous injection, and on the second day, the body weight decreased by about 6.4% and then was recovered to the body weight before injection as time went on. On the other hand, it was confirmed that no weight loss was observed in the case of the hydroxypropyl-gamma-cyclodextrin monomers and polymers. As verified in FIG. 23B, it was confirmed that the hydroxypropyl-beta-cyclodextrin groups showed the low behavioral score as they exhibited uneven hair, decreased activity, and decreased reaction rate on stimulation. On the other hand, the groups administered with PBS and hydroxypropyl-gamma-cyclodextrin monomers and polymers showed the highest behavioral score of 11 points for one week, but cyclodextrin hydroxypropyl-beta-cyclodextrin monomer showed the lowest behavioral score of an average of 6 points one day after cyclodextrin administration, and the score was gradually normalized.

As shown in FIG. 24, toxicity was not observed when tissues of the kidney, liver, spleen, heart, and lung were observed through a microscope.

As shown in FIGS. 25A and 25B, ototoxicity was measured by the viability of outer hair cells, and the higher viability appeared in the order of hydroxypropyl-beta-cyclodextrin (43.3 % survival), hydroxypropyl-gamma-cyclodextrin (74.7 % survival), and hydroxypropyl-gamma-cyclodextrin polymer (98 % survival), which confirmed that ototoxicity was significantly lowered. From this, it was confirmed that when the hydroxypropyl-gamma-cyclodextrin polymer is administered through systemic delivery, systemic toxicity and ototoxicity may be significantly reduced.

Also, as shown in FIG. 25C, when ototoxicity was observed after intraventricular injection, the higher viability appeared in the order of hydroxypropyl-beta-cyclodextrin (27.5% survival), hydroxypropyl-gamma-cyclodextrin (69.7% survival), and hydroxypropyl-gamma-cyclodextrin polymer (84.4% survival), which confirmed that ototoxicity was significantly lowered. Overall, theses results show that the ototoxicity may be significantly reduced when a hydroxypropyl-gamma-cyclodextrin polymer is administered through direct delivery to the central nervous system.

## Claims

1. A pharmaceutical composition for prevention or treatment of diseases related to cholesterol metabolism dysregulation, comprising a gamma-cyclodextrin (γ-cyclodextrin) polymer having a molecular weight in a range of about 2.5 kDa to about 50 kDa or a derivative thereof.

2. The pharmaceutical composition of claim 1, wherein the γ-cyclodextrin polymer comprises 2 to 35 γ-cyclodextrin monomers.

3. The pharmaceutical composition of claim 1, wherein the γ-cyclodextrin polymer is substituted with hydroxypropyl.

4. The pharmaceutical composition of claim 1, wherein the diseases related to cholesterol metabolism dysregulation are selected from the group consisting of Niemann-pick disease type C, Alzheimer's dementia, Parkinson's disease, focal segmental glomerulosclerosis (FSGS), Alport syndrome, diabetic kidney disease, multiple sclerosis, interstitial pneumonia, osteoarthritis, dyslipidemia, and cardiovascular diseases caused by cholesterol homeostasis dysregulation.

5. The pharmaceutical composition of claim 1, wherein the pharmaceutical composition is administered in combination with at least one selected from the group consisting of a liver X receptor (LXR) agonist, a GLP-1 receptor, an ezetimibe preparation, a fibric acid derivative, a nicotinic acid-based preparation, and an HMG-CoA reductase inhibitor.

6. The pharmaceutical composition of claim 5, wherein the LXR agonist is selected from the group consisting of 22(R)-hydroxycholesterol, 24(S),25-epoxycholesterol, 24(S)-hydroxycholesterol, 27-hydroxycholesterol, cholesteroic acid, T-314407, T-0901317, GW3965, SB742881, GSK 2186, WAY-252623 (LXR-623), WAY-254011, WYE-672, N,N-Dimethyl-3b-hydroxycholenamide (DMHCA), 4-(3-biaryl)quinoline sulfone, F₃MethylAA, acetyl-podocarpic dimer (APD), podocarpic imide dimer, CRX000541, CRX000864, CRX000929, CRX000823, CRX000987, CRX001093, CRX001094, CRX156651, CRX000909, CRX000908, CRX000369, CRX001045, CRX001046, LN7181, LN7179, LN7172, LN6672, LN7031, LN7033, LN6500, LN6662, Riccardin C, XL-652, DY136, DY142, and hypocholamide (3a,6a-dihydroxy-5b-cholanoic acid-N-methyl-N-methoxy-24-amide).

7. The pharmaceutical composition of claim 5, wherein the GLP-1 receptor is selected from the group consisting of exenatide, lixisenatide, and liraglutide.

8. The pharmaceutical composition of claim 5, wherein the HMG-CoA reductase inhibitor is selected from the group consisting of rosuvastatin, simvastatin, atorvastatin, pitavastatin, pravastatin, fluvastatin, and lovastatin.

9. The pharmaceutical composition of claim 5, wherein the fibric acid derivative is selected from the group consisting of gemfibrozil, bezafibrate, fenofibrate, and ciprofibrate.

10. A food composition for prevention or alleviation of diseases related to cholesterol metabolism dysregulation, comprising a γ-cyclodextrin polymer having a molecular weight in a range of about 2.5 kDa to about 50 kDa or a derivative thereof.

11. A health functional food composition for prevention or alleviation of diseases related to cholesterol metabolism dysregulation, comprising a γ-cyclodextrin polymer having a molecular weight in a range of about 2.5 kDa to about 50 kDa or a derivative thereof.

12. A method of preventing or alleviating diseases related to cholesterol metabolism dysregulation, the method comprising administering a γ-cyclodextrin polymer having a molecular weight in a range of about 2.5 kDa to about 50 kDa or a derivative thereof to a non-human subject.
